(19) Europäisches Patentamt
European Patent Office
Office européen des brevets

(11) Veröffentlichungsnummer: **0 206 219 B1**

(12) **EUROPÄISCHE PATENTSCHRIFT**

(45) Veröffentlichungstag der Patentschrift: **06.09.89**

(21) Anmeldenummer: **86108241.0**

(22) Anmeldetag: **16.06.86**

(51) Int. Cl.⁴: **A 61 K 31/41,** C 09 K 15/30, C 09 K 15/20, C 07 C 49/835, C 07 C 49/786, C 07 D 271/04, C 07 D 311/22

(54) **Photostabilisierung von Sydnoniminen.**

(30) Priorität: **21.06.85 DE 3522191**

(43) Veröffentlichungstag der Anmeldung: **30.12.86 Patentblatt 86/52**

(45) Bekanntmachung des Hinweises auf die Patenterteilung: **06.09.89 Patentblatt 89/36**

(84) Benannte Vertragsstaaten: **AT BE CH DE FR GB IT LI NL**

(56) Entgegenhaltungen:
**EP-A- 0 147 811**
**GB-A- 1 242 742**
**US-A- 4 271 031**

**CHEMICAL ABSTRACTS, Band 69, no. 5, 29 Juli 1968, Columbus, Ohio, USA, YASHUNSKII, V.G.; ZOTOVA, S.A. "Sydnones and sydnone imines. XL. Effect of substituents in the 4-position on the stability of the sydnone ring", p. 1723, Zusammenfassung no. 18 450f**
**CHEMICAL ABSTRACTS, Band 75, no. 13, 27 September 1971, Columbus, Ohio, USA, Y. ASAHI; K. SJINOZAKI; M. NAGAOKA "Chemical and kinetic study on stabilities of 3-morpholinosydnonimine and its N-ethoxycarbonyl derivative", p. 292, Zusammenfassung no. 87 742p**

(73) Patentinhaber: **CASSELLA Aktiengesellschaft, Hanauer Landstrasse 526, D-6000 Frankfurt am Main 61 (DE)**

(72) Erfinder: **Voegele, Dieter, Dr., Kurt-Schumacher-Ring 16, D-6454 Bruchköbel (DE)**
Erfinder: **Baumann, Petra, Rodaustrasse 33, D-6052 Mühlheim (DE)**
Erfinder: **Schönafinger, Karl, Dr., Holunderweg 8, D-8755 Alzenau (DE)**

(74) Vertreter: **Urbach, Hans-Georg, Dr., Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft die Stabilisierung von Sydnoniminen gegen Licht sowie Sydnonimine enthaltende Mischungen, die durch hohe Lichtstabilität ausgezeichnet sind und die als Lichtstabilisator eine Verbindung aus der Reihe der Flavonoide bzw. der korrespondierenden Hydroxychalcone («Index Nominum 1980», Centre scientifique de la Société suisse de pharmacie, Zürich (1979), Seite 881) enthalten.

Es ist eine seit langem bekannte und in vielen Bereichen der Technik häufig beobachtete Erscheinung, dass chemische Verbindungen durch Lichteinwirkung in ihrer Struktur verändert oder zerstört werden. Besonders bekannt ist das Ausbleichen von Farbstoffen und das Verspröden von Kunststoffen durch Licht. Besonders unangenehm und auch gefährlich sind die durch Licht ausgelösten chemischen Veränderungen in pharmakologisch wirksamen Substanzen. Hier kann die Lichteinwirkung zu einer Abschwächung der erwünschten Wirkung und/oder zum Auftreten neuer unter Umständen schädlicher Wirkungen führen. Wegen der damit verbundenen erheblichen Gefahren für den mit solchen pharmazeutischen Substanzen behandelten Patienten ist es von fundamentaler Bedeutung, diese pharmakologisch wirksamen, als Medikament eingesetzten Verbindungen gegen durch Licht ausgelöste chemische Veränderungen zu schützen.

Lichtschutz ist in vielen Bereichen der Technik erforderlich und wird auf verschiedenen Wegen praktiziert. So ist es bekannt, lichtempfindliche Stoffe in lichtundurchlässige Verpackungen einzuschliessen. Es ist auch bekannt, solche Umhüllungen einzusetzen, die teildurchlässig sind und die den Teil der Lichtstrahlung absorbieren, der die schädigende Wirkung entfaltet. Hierbei wird von der einleuchtenden Annahme Gebrauch gemacht, dass nur solche Strahlungsanteile zu einer chemischen Veränderung der Verbindung führen können, die von dieser Verbindung auch absorbiert werden. In diesem Sinne ist auch bereits vorgeschlagen worden, die zu schützenden Substanzen direkt mit dem Lichtschutzmittel zu überziehen. In die Gruppe dieser Ausführungsformen des Lichtschutzes fallen z.B. Lichtschutzanstriche aber auch kosmetische Zubereitungen mit Lichtschutzmitteln. Es ist auch bekannt, lichtempfindlichen Kunststoffen Lichtschutzmittel beizumengen und schliesslich ist, aufbauend auf Untersuchungen über die Photolyse des Nifedipins (Rainhard Klimek, Inaugural-Dissertation, Frankfurt (Main), 1978) auch vorgeschlagen worden, pharmakologisch wirksame Substanzen dadurch vor Lichtschädigung zu schützen, dass man sie mit Lösungen von lichtabsorbierenden Stoffen mischt und entweder in Form der lichtgeschützten Lösungen verwendet oder aus den Lösungen durch Entzug des Lösungsmittels feste, lichtstabilisierte Zubereitungen herstellt. Hierbei sollen als lichtabsorbierende Stoffe solche eingesetzt werden, die, insbesondere in dem stabilitätsgefährdeten Bereich von 300 bis 440 nm, ein ähnliches Absorptionsverhalten aufweisen wie dss zu schützende Pharmakon.

Dieser bekannte Vorschlag ist jedoch nicht verallgemeinerungsfähig. So ist es beispielsweise gezeigt worden, dass Nifedipin («Index Nominum 1980», Centre scientifique de la Société suisse de pharmacie, Zürich (1979), Seite 596) durch 8-Hydroxychinolin und durch gelbe Lebensmittelfarbstoffe, d.h. Verbindungen, die im Bereich der spectralen Absorption des Nifedpins ebenfalls starke Absorptionsbande aufweisen, sehr wirksam gegen Photolyse geschützt werden kann. Untersucht man dagegen den lichtinduzierten Abbau des Nifedipins in Gegenwart von Troxerutin, so zeigt sich kein nennenswerter Schutzeffekt, obgleich die Absorptionskurven dieser beiden Substanzen sich ebenfalls sehr ähneln und insbesondere in dem als besonders kritisch angesehenen Bereich von 300 bis 440 nm Absorptionsmaxima aufweisen, die nur ca. 10 nm auseinander liegen.

Hieraus schien sich zu ergeben, dass Troxerutin trotz theoretisch brauchbaren Absorptionsverhaltens aus anderen, bisher unbekannten Gründen zur Photostabilisierung von lichtempfindlichen Stoffen unbrauchbar ist.

Es war daher nicht vorherzusehen, dass Troxerutin und einige weitere Verbindungen aus der Reihe der Flavonole und Flavanone bzw. der Hydroxychalcone sich hervorragend zur Photostabilisierung von Molsidomin («Index Nominum 1980», Centre scientifique de la Société suisse de pharmacie, Zürich (1979), Seite 569) eignet. Dieser Befund ist um so überraschender als im Hinblick auf die bisherigen Vorstellungen über Lichtschutzwirkung Troxerutin das Molsidomin auch deshalb gar nicht schützen sollte, weil die Absorptionsmaxima dieses Substanzpaares im Bereich von 300 bis 440 nm viel weiter, nämlich ca. 40 nm, auseinander liegen als bei Nifedipin und Troxerutin.

Es ist daher bereits vorgeschlagen worden DE-A-3 346 638 und EP-A-0 147 811, diese Verbindungen zur Photostabilisierung von Molsidomin einzusetzen. Dieser Vorschlag beinhaltet auch die auf diesem Weige photostabilisierten Mischungen und pharmazeutischen Zubereitungen von Molsidomin.

Es wurde nun weiter gefunden, dass sich Flavonoide der Formel II

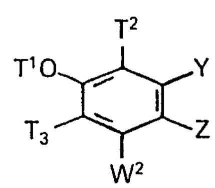

(II)

worin

$T^1$ Wasserstoff, Alkyl mit 1 oder 2 C-Atomen, β-Hydroxyethyl, oder einen Rest G,

$T^2$ Wasserstoff, Alkyl mit 1 oder 2 C-Atomen oder Alkanoyloxy mit 1 bis 4 C-Atomen,

$T^3$ Wasserstoff oder Alkyl mit 1 oder 2 C-Atomen,

Y eine Hydroxygruppe, Alkoxy mit 1 oder 2 C-Atomen oder β-Hydroxyethoxy und

Z eine Gruppe der Formel

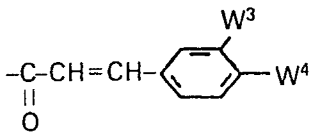

oder

Y und Z gemeinsam ein Strukturelement der Formeln III, IV oder V bedeuten,

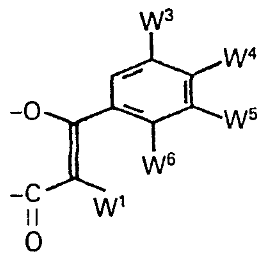

(III)

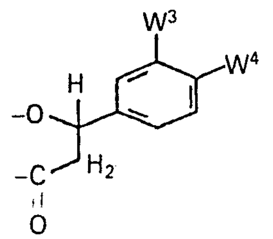

(IV)

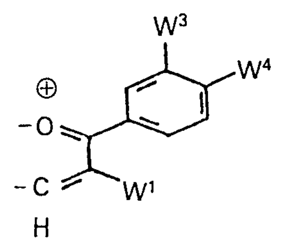

(V)

dessen freie Sauerstoffvalenz in der Y-Position und dessen freie Kohlenstoffvalenz in der Z-Position gebunden ist und

$W^1$ Wasserstoff oder eine der Gruppen –OH, Alkoxy mit 1 oder 2 C-Atomen oder –OG,

$W^2$ Wasserstoff oder eine der Gruppen –OH oder –OG,

$W^3$ Wasserstoff oder eine der Gruppen –OH, Alkoxy mit 1 oder 2 C-Atomen oder β-Hydroxyethoxy,

$W^4$ Wasserstoff oder eine der Gruppen –OH, Alkoxy mit 1 oder 2 C-Atomen oder β-Hydroxyethoxy,

$W^5$ Wasserstoff oder –OH,

$W^6$ Wasserstoff oder –OH und

G der Rest eines Mono- oder Disaccharids ist, und/oder einen Ester dieser Verbindungen mit Säuren sowie gegebenenfalls dessen Salz mit Basen sehr gut eignen, um Sydnonimine der Formel I

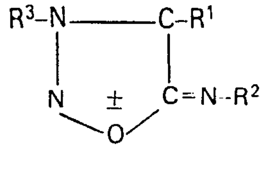

(I)

worin

$R^1$ Wasserstoff, Alkyl mit 1 bis 5 C-Atomen oder Halogen,

$R^2$ Wasserstoff, –NO, $-COR^4$ oder $-SO_2R^5$,

$R^3$ einen der Reste

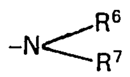 , 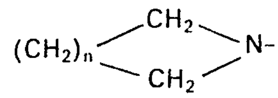 , 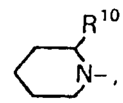 ,

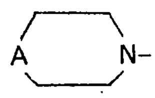 oder 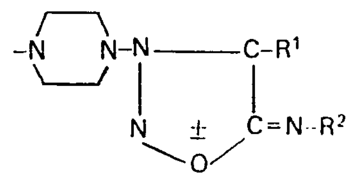

bedeuten, wobei

A für Sauerstoff oder eine der Gruppen $>S(O)_m$, $>N-R^{11}$, $>N-COOR^8$ oder $>N-SO_2R^9$ und

m für 0, 1 oder 2 und

n für 2, 3, 4, oder 5 stehen,

$R^4$ Wasserstoff, einen aliphatischen Rest mit 1 bis 6 C-Atomen, der auch durch Alkoxy mit 1 bis 6 C-Atomen oder Aryloxy mit 6 bis 12 C-Atomen substituiert sein kann, einen cycloaliphatischen Rest mit 5 bis 8 C-Atomen, einen bicycloaliphatischen Rest mit 7 bis 14 C-Atomen, einen tricycloaliphatischen Rest mit 7 bis 16 C-Atomen, Aryl mit 6 bis 12 C-Atomen, das auch durch Halogen und/oder Alkyl mit 1 bis 4 C-Atomen und/oder Alkoxi mit 1 bis 4 C-Atomen mono-, di- oder trisubstituiert sein kann, Aralkyl oder Aralkenyl mit 7 bis 13 C-Atomen, Alkoxy mit 1 bis 6 C-Atomen, Cycloalkoxy mit 5 bis 8 C-Atomen, Bicycloalkoxy mit 7 bis 12 C-Atomen, Tricycloalkoxy mit 7 bis 16 C-Atomen, Aryloxy mit 6 bis 12 C-Atomen, Pyridyl, Pyrrolyl, Imidazolyl, Pyrazolyl, Pyrazinyl, Pyrimidinyl, Pyridazinyl, Alkoxicarbonyl mit insgesamt 2 bis 7 C-Atomen,

$R^5$ Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem der Alkylreste,

$R^6$ und $R^7$ unabhängig voneinander Alkyl mit 1 bis 6 C-Atomen, Alkenyl mit 3 bis 6 C-Atomen, Cycloalkyl mit 5 bis 8 C-Atomen, Bicycloalkyl mit 7 bis 14 C-Atomen, Tricycloalkyl mit 7 bis 16 C-Atomen, Aralkyl mit 7 bis 9 C-Atomen, wobei die Aralkylgruppe durch Methyl oder Halogen substituiert sein kann, oder einen über eine Alkylenkette an das Stickstoffatom der 3-Aminogruppe gebundenen heterocyclischen Ring bedeuten und

$R^6$ zusätzlich Wasserstoff und

$R^7$ zusätzlich ein Rest der Formel 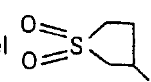

sein kann,

$R^8$ Alkyl mit 1 bis 4 C-Atomen,

$R^9$ Alkyl mit 1 bis 6 C-Atomen, Aryl mit 6 bis 12 C-Atomen, das auch durch Methyl oder Chlor

substituiert sein kann oder eine Dialkylaminogruppe mit 1 bis 4 C-Atomen in jedem der Alkylreste,

$R^{10}$ Alkyl mit 1 bis 4 C-Atomen und

$R^{11}$ Alkyl mit 1 bis 6 C-Atomen bedeuten,

oder deren Salze zu photostabilisieren.

Die vorliegende Erfindung betrifft auch photostabilisierte Mischungen von Sydnoniminen der Formel I, einem oder mehreren Photostabilisatoren der Formel II und gegebenenfalls weiteren Mischungskomponenten, wie z.B. Lösungsmitteln, Dispergiermitteln, Hilfs- und Zusatzstoffen. Insbesondere betrifft die vorliegende Erfindung auch photostabilisierte pharmazeutische Zubereitungen dieser Sydnonimine, die als Photostabilisator eine oder mehrere Verbindungen der Formel II sowie weitere in der Galenik übliche Hilfs- und Zusatzstoffe enthalten.

Die Verbindungen der Formel II zeigen alle, ähnlich dem Troxerutin, eine überraschende, hohe Lichtschutzwirkung für die Sydnonimine, die im Hinblick auf die relativ schwache Lichtabsorption dieser Verbindungen in keiner Weise zu erwarten war.

In den Photostabilisatoren der Formel II ist $T^2$ vorzugsweise nur dann ungleich Wasserstoff, wenn Y und Z gemeinsam einen Rest der Formel IV bilden, wobei dann $T^2$ vorzugsweise ein Alkylrest mit 1 oder 2 C-Atomen ist, oder wenn Y und Z gemeinsam einen Rest der Formel III mit $W^1$=OH bilden, d.h. bei Flavonolderivaten, wobei dann $T^2$ vorzugsweise ein Alkanoyloxyrest mit 1 bis 4 C-Atomen ist.

$T^3$ ist vorzugsweise nur dann Alkyl mit 1 oder 2 C-Atomen, wenn Y und Z gemeinsam einen Rest der Formel IV bilden. Vorzugsweise nur, wenn Y und Z gemeinsam einen Rest der Formel III mit $W^1$=OH bilden, können $W^2$ Wasserstoff und $W^5$ und $W^6$ unabhängig voneinander –OH bedeuten.

Bevorzugt sind solche Photostabilisatoren der Formel II, in denen Y und Z gemeinsam einen Rest der Formel III oder IV bilden.

Bilden Y und Z gemeinsam einen Rest der Formel V, so stehen $W^1$ und $W^2$ unabhängig voneinander vorzugsweise für –OH und insbesondere für –OG, $T^1$, $T^2$ und $T^3$ für Wasserstoff und $W^3$ und $W^4$ für OH.

Steht Z für den obigen Cinnamoylrest der Formel

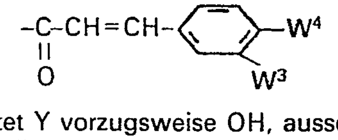

so bedeutet Y vorzugsweise OH, ausserdem bedeuten in diesem Fall vorzugsweise $W^3$ und $W^4$, mindestens aber $W^4$, OH oder Alkoxy und mindestens eines der Symbole $W^3$ oder $W^4$ OH; weitere bevorzugte Verbindungen aus dieser Klasse sind solche, in denen $W^2$ ein Rest –OG und $T^2$ und $T^3$ Wasserstoff sind.

Sind Y und Z gemeinsam ein Rest der Formel IV, so gelten unabhängig voneinander folgende bevorzugte Merkmale: $W^2$ ist OH; $T^1$ ist H oder G; $W^4$ ist OH oder Alkoxy mit 1 oder 2 C-Atomen; $T^2$ und $T^3$ sind Wasserstoff.

Sind Y und Z gemeinsam ein Rest der Formel III, in dem $W^1$ ungleich Wasserstoff ist, so gelten unabhängig voneinander folgende bevorzugte Merkmale: $W^2$ ist OH und $T^2$ und $T^3$ sind Wasserstoff.

Bevorzugt für den erfindungsgemässen Einsatz sind ferner solche Verbindungen der Formel II, in denen Y, Z, $W^1$ und G die oben genannten Bedeutungen haben, $W^4$ eine der Gruppen OH, Alkoxy mit 1 oder 2 C-Atomen oder β-Hydroxyethoxy und $W^3$ –OH oder β-Hydroxyethoxy und $T^1$ Wasserstoff, β-Hydroxyethyl oder ein Rest G eines Mono- oder Disaccharids und $T^2$ und $T^3$ Wasserstoff sind, insbesondere dann, wenn Y und Z gemeinsam einen Rest der Formel III bilden. Bevorzugt sind auch solche Verbindungen der Formel II, in denen $W^1$ einen Rest –OG oder $T^1$ einen Rest G bedeutet.

Besonders bevorzugt sind solche Verbindungen der Formel II, die mehrere bevorzugte Merkmale aufweisen. Besonders bevorzugt sind beispielsweise Hesperidinmethyl-chalcon, Hesperidin-phosphat, Hesperidin, Hesperetin, Quercetin und seine Derivate wie z.B. Quercitrin, Rutin, Rutin-sulfat, Naringenin, Kämpferol und seine Derivate, insbesondere sein 7,4'-Dimethylether, Morin, Apigetrin, Luteolin und dessen 7-Glucosid, und insbesondere Troxerutin.

Soweit die Verbindungen der Formel II freie OH-Gruppen aufweisen, können diese mit vorzugsweise pharmazeutisch unbedenklichen Säuren verestert werden. Zur Veresterung geeignete Säuren sind z.B. niedere aliphatische Carbonsäuren, wie Ameisensäure, Essigsäure, Propionsäure, Buttersäure, Milchsäure, Maleinsäure, Bernsteinsäure, Apfelsäure, Weinsäure oder anorganische Säure, wie Schwefelsäure und insbesondere Phosphorsäure. Bei der Veresterung mit mehrbasischen Säuren werden die 1:1-Veresterungsprodukte hergestellt, bei denen jeweils nur eine der Säurefunktionen verestert, die übrigen frei sind. Bevorzugte Ester sind solche, die in der Molsidomin-Mischung eine bessere Löslichkeit haben als die unveresterte Verbindung.

Sowohl die schwach sauren aromatischen Hydroxylgruppen der erfindungsgemässen Verbindungen der Formel II als auch die freien Säurefunktionen der sauren Ester der Verbindungen der Formel II mit mehrbasischen Säuren können durch Einwirkung von pharmakologisch unbedenklichen Basen in die Salze überführt werden. Auch diese Salze zeigen die hohe Lichtschutzwirkung der Verbindungen der Formel II und sind daher zum erfindungsgemässen Einsatz gut geeignet. Sie können gegenüber den unveresterten Verbindungen der Formel II auch noch anwendungstechnische Vorteile aufweisen. Wenn z.B. die freie Verbindung der Formel II aufgrund geringer Löslichkeit schlecht in eine wässrige Zubereitung eingearbeitet werden kann, so kann der Einsatz eines Salzes mit erhöhter Wasserlöslichkeit, wie z.B. eines Alkalisalzes eines Phosphorsäuremonoesters, die Herstellung der Zubereitung erheblich erleichtern.

Die Verbindungen der Formel II können einzeln

oder auch im Gemisch untereinander für den erfindungsgemässen Zweck eingesetzt werden. Sofern in bestimmten galenischen Zubereitungen eine zu geringe Löslichkeit der erfindungsgemäss einzusetzenden Photostabilisatoren vorliegt, kann auch der Zusatz von Lösungsvermittlern und solubilisierend wirkenden Hilfsstoffen zweckmässig sein. Solche, die gleichmässige Verteilung von schwerlöslichen Komponenten in pharmazeutischen Zubereitungen begünstigende bzw. bewirkende Mittel sind aus der Literatur (z.B. H. Sukker. P. Fuchs. P. Speiser, Pharmazeutische Technologie, (1978) G. Thieme Verlag, Stuttgart, Seite 281 ff.) in grosser Zahl bekannt. Beispiele für solche Hilfsstoffe sind polyoxethlierte Fettalkoholether, polyoxethylierte Sorbitan-Fettsäureester oder polyoxethylierte Stearinsäurepolyolester.

In den photostabilisierten Sydnoniminen der Formel I steht $R^1$ vorzugsweise für Wasserstoff. Für den Fall, dass $R^1$ für Halogen steht, ist Chlor oder Brom bevorzugt. Die für $R^4$ stehenden aliphatischen Reste, Alkoxireste oder Alkoxicarbonylreste können geradkettig oder verzweigt sein. Als für $R^4$ stehende aliphatische Reste kommen insbesondere Alkylreste mit 1 bis 4 C-Atomen in Betracht. Falls das für $R^4$ stehende Alkyl durch Alkoxi substituiert ist, besitzen diese Alkoxireste vorzugsweise 1 bis 4 C-Atome; insbesondere ist hier der Methoximethylrest zu nennen. Als für $R^4$ stehende aliphatische Reste, die durch Alkoxi mit 1 bis 3 C-Atomen substituiert sind, ist inbesondere der Methoxi-methyl-rest zu nennen. Als für $R^4$ stehende cycloaliphatische Reste kommen vor allem Cycloalkylreste mit 5 bis 7 C-Atomen, insbesondere Cyclopentyl, und bevorzugt Cyclohexyl in Betracht. Als für $R^4$ stehender bicycloaliphatischer Rest kommt insbesondere das 2.6.6-Trimethylbicyclo(3.1.1)-heptan-3-yl(=Pinanyl) in Betracht. Als für $R^4$ stehender tricycloaliphatischer Rest kommt insbesondere das Tricyclo- ($3.3.1.1^{3,7}$) -decan-1-yl (=Adamantanyl) in Betracht. Als für $R^4$ stehende Alkoxireste kommen insbesondere Methoxi- und Ethoxi-Reste in Betracht. Als für $R^4$ stehender Alkoxicarbonylrest kommt insbesondere der Ethoxicarbonyl-rest in Betracht. Als für $R^4$ stehende Arylreste sind z.B. α- oder β-Naphthylreste, insbesondere aber der Phenylrest, zu nennen. Als für $R^4$ stehende Aryloxireste sind z.B. α- oder β-Naphthoxi-Reste, insbesondere aber der Phenoxi-Rest, zu nennen. Die für $R^4$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Als Halogensubstituenten für die Arylreste kommen z.B. Chlor und Bromatome in Betracht.

Als Arylrest wird für $R^4$ der Phenylrest bevorzugt, der unsubstituiert oder durch Chlor, Methyl und/oder Methoxi oder Ethoxi ein-, zwei- oder dreifach substituiert sein kann.

Als für $R^4$ stehende substituierte Arylreste sind insbesondere zu nennen: Methyl-phenyl(Tolyl), Nitro-phenyl und Chlor-phenyl.

Als Arylalkyl-Rest werden für $R^4$ Benzyl und Phenethyl und als Arylalkenyl-Rest Styryl bevorzugt. Weitere bevorzugte Reste sind:
$R^2$ = H, insbesondere, wenn $R^1$ = H und/oder A=$>$ $SO_2$ ist, sowie $R^4$ = Alkoxi mit 1 oder 2 C-Atomen oder Alkoxicarbonyl mit insgesamt 2 oder 3 C-Atomen. Die für $R^5$ und/oder $R^9$ stehenden Alkylreste sind vorzugsweise Methylreste. Die für $R^5$ und/oder $R^9$ stehenden Arylreste sind insbesondere Phenylreste, die insbesondere auch durch Methyl oder Chlor in 4-Stellung substituiert sein können. Die für $R^5$ und/oder $R^9$ stehenden Dialkylaminoreste besitzen insbesondere in jeder Alkylgruppe 1 oder 2 C-Atome.

Von den für $R^4$ stehenden heteroaromatischen Pyrrolyl-, Imidazolyl-, Pyrazolyl-, Pyrazinyl-, Pyrimidinyl-, Pyridazinyl- und Pyridyl-Resten ist der Pyridyl-Rest bevorzugt.

Für $R^8$ sind Ethyl und Methyl bevorzugt. Für $R^4$ sind bevorzugt: Methyl, Ethyl, Cyclohexyl, Phenyl, 4-Chlor-phenyl und 4-Nitrophenyl.

Für $R^3$ ist der Rest

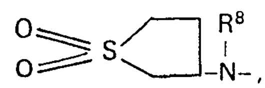

insbesondere mit $R^8$ = Methyl, sowie insbesondere in Kombination mit $R^2$ = Wasserstoff, bevorzugt.

Bevorzugt werden erfindungsgemäss stabilisierte Sydnonimine der Formel I, worin
$R^2$ Wasserstoff oder $5COR^4$,
$R^3$ einen der Reste

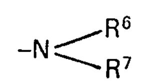

oder

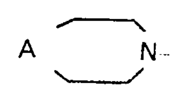

bedeuten, wobei

A für Sauerstoff oder eine der Gruppen $>S(O)_m$, $>N-COOR^8$ oder $>N-SO_2R^9$ und

m für 0, 1 oder 2 stehen,

$R^4$ Wasserstoff, Alkyl mit 1 bis 4 C-Atomen, das auch substituiert sein kann, Cycloalkyl mit 5 bis 8 C-Atomen, Bicycloalkyl mit 7 bis 12 C-Atomen, Phenyl, das auch durch Halogen substituiert sein kann, Alkoxy mit 1 bis 4 C-Atomen, Cycloalkoxy mit 5 bis 8 C-Atomen,

$R^6$ Wasserstoff oder Alkyl mit 1 bis 4 C-Atomen,

$R^7$ einen Rest der Formel 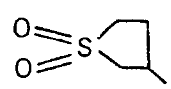

$R^8$ Alkyl mit 1 bis 4 C-Atomen und

$R^9$ Alkyl mit 1 bis 4 C-Atomen oder eine Dialkylaminogruppe mit 1 bis 4 C-Atomen in jedem der Alkylreste bedeuten.

Beispiele für Bedeutungen von $R^2$ bevorzugter Sydnonimine sind:
Wasserstoff, Methoxycarbonyl, Ethoxycarbonyl, Cyclohexoxycarbonyl, Phenoxycarbonyl, Benzoyl, Chlorbenzoyl, Formyl, Acetyl, Propionyl, Butyryl, Methoxyacetyl, Ethoxyacetyl, Phenoxyacetyl, Cyclohexylcarbonyl, Pinanylcarbonyl.

Beispiele für Reste $R^3$ bevorzugter Sydnonimine sind:

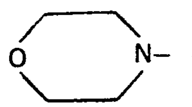 , 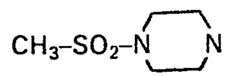 ,

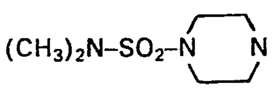 , 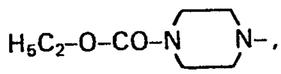 ,

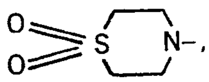 , 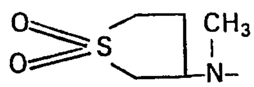

Besonders bevorzugte Sydnonimine der Formel I sind solche, die mehrere bevorzugte Merkmale aufweisen. Entsprechend sind solche erfindungsgemässen photostabilisierten Sydnonimin-Mischungen besonders bevorzugt, die bevorzugte Sydnonimine der Formel I und bevorzugte Flavonoide der Formel II enthalten.

Die erfindungsgemässen stabilisierten Sydnonimine und ihre Herstellung sind bekannt (vergl. DE-A-16 70 127, 17 70 061, 10 42 854, 31 07 933, DE-B-16 95 897, FR-A-2 150 306, EP-B-23 343).

Die erfindungsgemäss einzusetzenden Verbindungen der Formel II sind entweder bekannt und zum Teil handelsüblich, oder sie lassen sich vom Fachmann durch einfache, bekannte Alkylierungs- oder Hydroxyethylierungsreaktionen oder durch hydrolytische Abspaltung eines Saccharidrestes aus bekannten Verbindungen herstellen. Es sind auch verschiedene gute Methoden bekannt zur Herstellung dieser Verbindungen, ausgehend von Resorcin und Resorcinderivaten (vgl. hierzu die Lehrbücher der organischen Chemie z.B. Gabor Fodor, Organische Chemie, Bd. 2, VEB Deutscher Verlag der Wissenschaften, Berlin (1965); L.F. Fieser und M. Fieser, Lehrbuch der Organischen Chemie, Verlag Chemie GmbH, Weinheim, Bergstrasse (1957) ).

So können die für $T^1$ stehenden und die in $W^1$, $W^3$ und $W^4$ enthaltenen Niederalkylgruppen durch Umsetzung der korrespondierenden phenolischen Verbindungen in schwach alkalischem, vorzugsweise wässrigem Medium mit bekannten Methylierungs- oder Ethylierungsmitteln, wie z.B. Dimethyl- oder Diethylsulfat, erhalten werden. Die entsprechenden Hydroxyethyl-Derivate können analog durch Umsetzung der phenolischen Verbindungen mit Ethylenoxid hergestellt werden. Eventuell anfallende Reaktionsgemische können durch bekannte Methoden, z.B. fraktioniertes Kristallisieren, vorzugsweise durch Chromatographie, getrennt werden; sie können aber ebensogut als Mischung für den erfindungsgemässen Zweck eingesetzt werden. Sofern eine Synthese, ausgehend von Resorcin oder seinen Derivaten, gewünscht wird, kann durch Umsetzung der Ausgangsverbindung zum Trihydroxy- oder Trialkoxy-acetophenon und dessen Kondensation mit Dialkoxybenzaldehyd das gegebenenfalls entsprechend alkylierte Chalcon der Formel II dargestellt werden. Das freie Chalcon kann gewünschtenfalls durch Protonenkatalyse zum entsprechend substituierten Flavononderivat der Formel I cyclisiert werden, und dieses wiederum gestattet, z.B. durch Umsetzung mit salpetriger Säure und anschliessende Hydrolyse den Zugang in die Reihe der Flavone der Formel II.

Überraschenderweise ist die Lichtschutzwirkung von Verbindungen der Formel II für Sydnonimine erheblich besser als die des als Lichtschutzmittel ebenfalls bereits bekannten Lebensmittelfarbstoffs Chinolingelb («Richtlinie für färbende Stoffe» vom 23.10.1962, E 104; Chinophthalon-sulfonsäure). So ergibt sich z.B. für eine Lösung von 2 mg/ml Molsidomin in 0,9%iger NaCl-Lösung ohne Lichtschutzzusatz und Standard-Belichtung ein Regressionskoeffizient von −0,165, in Gegenwart von 10 mg/ml Kämpferol-7,4'-dimethylether ein Regressionskoeffizient von 0,0181 und in Gegenwart von 10 mg/ml Chinolingelb ein Regressionskoeffizient von −0,089.

Auch eine Messung des Molsidominabbaus in einer Lösung der Substanz in 0,9%iger Kochsalzlösung mit und ohne Zusatz eines Photostabilisators der Formel II zeigt dessen nicht vorhersehbare ausgezeichnete Lichtschutzwirkung: So ergibt sich bei Belichtung einer solchen Molsidominlösung bei einer Anfangskonzentration von 2 mg/ml Molsidomin unter natürlichen Belichtungsbedingungen ohne Photostabilisator ein Regressionskoeffizient des Molsidominabbaus von −6,34, in Anwesenheit von 18 mg/ml von Apigenin-7-glucosid unter gleichen Belichtungsbedingungen ein Regressionskoeffizient von −1,0.

Die hohe Schutzwirkung von Troxerutin zeigt sich auch bei Belichtung einer Tablettenzubereitung von 3-(4-Methylsulfonylpiperazin-1-yl)-N-ethoxycarbonyl-sydnonimin unter standardisierten Belichtungsbedingungen (Suntest). Ohne Troxerutin wird das Sydnonimin in der Tablette (8 mg) im Verlauf von 6 h auf 33,5% der ursprünglichen Menge abgebaut, während bereits bei einem Zusatz von nur 8 mg Troxerutin der Abbau so verlangsamt wird, dass nach 6 h noch 90,3% des Wirkstoffs erhalten sind und bei Einsatz von 80 mg Troxerutin praktisch kein merklicher Wirkstoffabbau mehr erfolgt.

Wird die Konzentration der Verbindungen der Formel II in Lösungen oder anderen Zubereitungen der Sydnonimine der Formel I schrittweise herabgesetzt und jeweils unter gleichen Belichtungsbedingungen der Sydnoniminabbau gemessen, so findet man überraschenderweise, dass die Lichtschutzwirkung mit der Konzentration des Photostabilisators der Formel II nur sehr langsam zurückgeht.

Ein Beispiel hierfür ist der belichtungsbedingte Abbau von Molsidomin in Retardtabletten mit 8 mg Wirkstoffgehalt ohne und in Gegenwart von 8 mg, 0,8 mg und 0,08 mg Luteolin pro Tablette. Die Messung zeigt, dass unter der Lichteinwirkung nach einer Anfangsphase, in der eine sprunghafte Abnahme der Molsidominmenge in

der Tablette erfolgt, ein sehr gut linearer Abbau des Wirkstoffs eintritt, wobei die Molsidominmenge in der luteolinfreien Tablette bereits nach 4,4 Std. auf 50% der Anfangsmenge gefallen ist, während bei der Tablette, die Luteolin in einer Menge von nur 1/100 der Wirkstoffmenge enthält, erst nach 9,6 Std. 50% des Wirkstoffs abgebaut sind. Hieraus ergibt sich, dass schon durch einen Zusatz von Molsidomin in einer Menge von 1/100 der Wirkstoffmenge sich in Stabilität des Molsidomins in den Tabletten um den Faktor 2,2 erhöht.

Noch drastischer ist die Verbesserung der Lichtstabilität von Molsidomin durch Zusatz von Kämpferol-7,4'-dimethylether als Photostabilisator in flüssigen Zubereitungen zu erkennen. Misst man z.B. die Wirkstoffabnahme von Molsidomin-Tropfen, die 1,8 mg Molsidomin und den Photostabilisator in wechselnden Mengen zwischen 18 mg und 0,0018 (!) mg pro ml enthalten und die in Braunglasflaschen abgefüllt sind, in einem Suntest-Gerät, so ergibt sich, dass nach 15 Stunden Belichtungsdauer folgende Molsidominkonzentrationen angetroffen werden:

a) ohne Photostabilisator 20%
b) mit 18 mg/ml Photostabilisator: > 99%
c) mit 1,8 mg/ml Photostabilisator 96%
d) mit 0,18 mg/ml Photostabilisator 87%
e) mit 0,0018 mg/ml Photostabilisator 73%.

Dies entspricht einer Stabilitätsverbesserung selbst bei der niedrigsten Konzentration des Photostabilisators um den Faktor 3,6.

Zur Photostabilisierung werden die Verbindungen der Formel II dem Sydonimin im Mengenverhältnis 0,001:1 bis 50:1, vorzugsweise 0,1:1 bis 20:1 zugesetzt. In der Praxis, um eine Schutzreserve auch für ungünstige Belichtungsverhältnisse zu schaffen, kann es zweckmässig sein, als Untergrenze dem Sydnonimin die 0,3- bis 0,5fache Menge an Photostabilisator der Formel II zuzusetzen.

Eine Herabsetzung der Stabilisatormenge unter 1/1000 der Sydoniminmenge hat eine kontinuierliche Verminderung der Schutzwirkung zur Folge; sie hat dort ihre Grenze, wo die Schutzwirkung nicht mehr den Anforderungen gerecht wird. Noch niedrigere Stabilisatorzusätze sind daher als eine verschlechterte Ausführungsform der vorliegenden Erfindung zu betrachten. Das gleiche gilt für Zusätze, die deutlich über der oben angegebenen Höchstgrenze praktischen Nutzen für die Stabilisierung gegenüber Licht.

In Sydnonimin-Mischungen oder pharmazeutischen Sydnonimin-Zubereitungen, die sehr niedrige Konzentrationen von Sydnonimin enthalten, wird man zweckmässigerweise nicht nur 1/1000 der Sydnonimin-Menge an Photostabilisatoren der Formel II zusetzen, da dies zu technisch schwer zu realisierenden Stabilisatorkonzentrationen führen würde und ein unzureichender Lichtschutz zu befürchten ist. Vorteilhafterweise werden daher in der Praxis den Sydnonimin-Mischungen und -Zubereitungen Photostabilisatoren in einer Menge zugesetzt, die mindestens der 0,1fachen, vorzugsweise mindestens der 0,3fachen und insbesondere mindestens der 0,5fachen Menge der Sydnonimins entsprechen. In der Regel ergeben sich hierbei in der Praxis Stabilisatorkonzentrationen von mindestens 0,062 bis 0,5 Gew.-%, vorzugsweise 0,19 bis 1,5 Gew.-%, insbesondere 0,31 bis 2,5 Gew.-%, bezogen auf das Gewicht der Gesamtmischung.

Die Photostabilisierung der reinen Sydnonimine durch Zusatz einer oder mehrerer Verbindungen der Formel II, d.h. die Herstellung von Mischungen, die ausser dem Sydnonimin und dem Stabilisator keine weiteren Zusätze enthalten, kommt insbesondere dann in Betracht, wenn es gilt, das Sydnonimin bei der Herstellung, beim Versand, bei der Lagerung und bei der Weiterverarbeitung gegen Licht zu schützen.

Selbstverständlich können die Sydnonimine auch für – nicht-pharmazeutische Zwecke durch Zusatz von Verbindungen der Formel II stabilisiert werden. Je nach dem technischen Einsatzgebiet können dann neben dem Sydnonimin und der Verbindung der Formel II auch noch andere Hilfs- oder Zusatzstoffe, wie Träger-, Netz- oder Dispergiermittel, evtl. auch Nahrungsstoffe, in den Mischungen enthalten sein.

Auch für den pharmazeutischen Einsatz werden in der Regel nicht die photostabilen Mischungen verwendet, die ausschliesslich Sydnonimin und eine oder mehrere Verbindungen der Formel II enthalten, sondern es werden entweder Lösungsmittel oder aber feste Füll-, Verdünnungs- oder Hilfsmittel, wie sie bei der Herstellung pharmazeutischer Zubereitungen in der Galenik üblich sind, in den Mischungen enthalten sein.

Die erfindungsgemässen photostabilisierten Sydnonimin-Zubereitungen können Wirkstoff und Photostabilisator der Formel II in fester und/ oder gelöster Form enthalten.

Erfindungsgemäss durch Verbindungen der Formel II lichtstabilisierte pharmazeutische Zubereitungen können flüssig bis fest sein. Flüssige pharmazeutische Zubereitungen sind z.B. Lösungen wie sie für Injection oder Infusion oder für orale Applikation in Form von Tropfen eingesetzt werden. Solche erfindungsgemässen Lösungen enthalten das Sydnonimin in Konzentrationen, die in der Regel je nach Applikationsart zwischen 0,01 mg/ml und 100 mg/ml liegen. Neben dem Sydnonimin enthalten die Lösungen die erforderliche Konzentration an Verbindungen der Formel II, die, wie oben angegeben, das 0,001- bis 50fache der Sydnoniminkonzentration beträgt, sowie das Lösungsmittel, wie z.B. isotonische Kochsalzlösung, Wasser, andere übliche verträgliche Lösungsmittel bzw. Lösungsmittelgemische und gegebenenfalls weitere in der Galenik übliche Hilfsstoffe, oder, sofern es sich um ein Kombinationspräparat handelt, auch weitere pharmazeutische Wirkstoffe. Verdickte Lösungen, wie z.B. Pasten, Salben oder Pflaster, die zur transdermalen Therapie geeignet sind, können auch noch hohere Sydnoniminkonzentrationen aufweisen.

Feste Zubereitungen enthalten das Sydnonimin in Konzentrationen, die in der Regel je nach Zubereitungsform zwischen 0,1 mg/g und 50 mg/g

liegen, die erforderliche Menge an Verbindungen der Formel II, die wie oben angegeben, das 0,001-, vorzugsweise 0,3- bis 50fache, insbesondere das 0,5 bis 20fache der Sydnoniminmenge beträgt, sowie die in galenischen Zubereitungen üblichen weiteren Zusätze, z.B. von Füllmitteln, Quellmitteln, Sprengmitteln, Gleitmitteln, Haftmitteln, Form- und Trennmitteln, Überzugsmitteln oder Kapselmaterialien usw., wie sie aus der Literatur bekannt sind, oder, sofern es sich um ein Kombinationspräparat handelt, auch weitere pharmazeutische Wirkstoffe.

Bei festen geformten Zubereitungen kann der erfindungsgemässe Lichtschutz auch dadurch erfolgen, dass man eine feste geformte Sydnonimin-Zubereitung, die frei ist von Lichtschutzmitteln, mit einem Überzug versieht, der eine ausreichende Konzentration einer Verbindung der Formel I enthält.

Dieses Verfahren kommt vor allem für Tabletten, Pillen und Dragees in Betracht. Die erforderliche Konzentration des Photostabilisators in dem Überzug hängt vorwiegend von der normalerweise zu erwartenden Lichtbelastung des Präparates ab. In der Regel wird die Konzentration der Verbindungen der Formel II in dem Überzug zwischen 2 und 30 Gew.-%, vorzugsweise 10 bis 25 Gew.-% bezogen auf die Überzugsmasse, gewählt.

Die für das konkrete Anwendungsgebiet geeignete Sydnoniminkonzentration kann in an sich bekannter Weise aus den Wirkungsdaten der Substanz leicht berechnet werden. Sie liegen z.B. beim Molsidomin für Infusionslösungen bei 0,002 mg/ml bis 0,4 mg/ml, für Injektionslösungen bei 0,2 mg/ml bis 20 mg/ml, für Tropfen bei 0,4 mg/ml bis 20 mg/ml, für Tabletten, überzogene Tabletten und Kapseln bei 5 mg/ml bis 200 mg/g.

Die Herstellung fester bis flüssiger pharmazeutischer Zubereitungen ist an sich bekannt durch eine umfassende Literatur. Die Herstellung der erfindungsgemässen, lichtstabilisierten Sydnonimin-Zubereitungen kann nach diesen, an sich bekannten Methoden erfolgen. Hervorzuheben ist lediglich, dass auch bei der Herstellung fester Zubereitungen alle bekannten Verfahren eingesetzt werden können. So ist es möglich aber nicht erforderlich, die Zubereitung durch Eindampfen einer Lösung der Bestandteile oder einer Mischung einiger fester Bestandteile und einer Lösung einer oder mehrerer Verbindungen der Formel II herzustellen. Die erfindungsgemässe Lichtschutzwirkung ergibt sich aber überraschenderweise auch, wenn alle Bestandteile in fester Form zusammengebracht und in bekannter Weise zu einer pharmazeutischen Zubereitung verarbeitet werden.

Der Einsatz von Verbindungen der Formel II als Lichtschutzmittel für Sydnonimin ist von besonderem Vorteil, weil die Schutzwirkung sehr hoch ist, man also mit geringen Zusatzmengen einen ausreichenden Lichtschutz erhält. Gegenüber dem grundsätzlich auch möglichen Lichtschutz durch Lebensmittelfarbstoffe bietet der Einsatz von Verbindungen der Formel II neben der hohen Wirksamkeit jedoch noch einen zusätzlichen Vorteil:

Lebensmittelfarbstoffe müssen, wenn sie einen wirksamen Lichtschutz bieten sollen, in erheblich höherer Konzentration eingesetzt werden als sie üblicherweise für das Anfärben von Lebensmitteln verwendet werden. Da Lebensmittelfarbstoffe aber auch im Tagesverlauf mit den verschiedensten Nahrungsmitteln in nicht kontrollierten Mengen aufgenommen werden könnte bei zusätzlicher Zufuhr in Arzneimitteln evtl. eine Annäherung oder gar eine Überschreitung der empfohlenen Tages-Höchstdosis dieser Farbstoffe erfolgen. Diese Gefahr wird bei Einsatz der Flavonoide der Formel II als Lichtschutzstoff vermieden.

Zur Veranschaulichung der Erfindung seien die folgenden Ausführungsbeispiele angegeben.

Beispiel 1

Erfindungsgemässe, lichtstabilisierte Molsidomintropfen haben beispielsweise folgende Zusammensetzung:

| | |
|---|---|
| Molsidomin | 2 mg |
| Luteolin | 15 mg |
| Methylparaben | 0,7 mg |
| Propylparaben | 0,3 mg |
| entmineralisiertes Wasser/Ethanol (1:1) ad | 1 ml |

Luteolin:

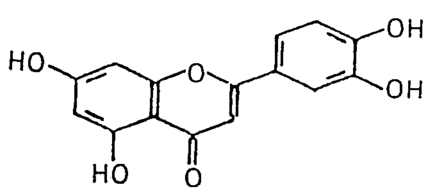

Herstellung

In 4 Liter einer Mischung von entmineralisiertem Wasser/Ethanol 1:1 (Vol.-Teile) werden 3,5 g Methylparaben und 1,5 g Propylparaben gelöst; anschliessend werden in dieser Lösung 10 g Molsidomin und 75 g Luteolin unter Rühren aufgelöst und das Volumen der Lösung durch Zusatz von entmineralisiertem Wasser/Ethanol (1:1) auf 5 Liter eingestellt.

Beispiel 2

Eine erfindungsgemässe, lichtstabilisierte Lösung von 3-(4-Methylsulfonylpiperazin-1-yl)-N-ethoxycarbonylsydnonimin für Ampullen bzw. Infusionsampullen hat beispielsweise folgende Zusammensetzung:

| | |
|---|---|
| 3-(4-Methylsulfonylpiperazin-1-yl)-N-ethoxycarbonylsydnonimin | 2 mg |
| Troxerutin | 2 mg |
| Wasser für Injektionszwecke ad | 1 ml |

Herstellung

In 3 Liter Wasser für Injektionszwecke werden

10 g 3-(4-Methylsulfonylpiperazin-1-yl)- N-ethoxycarbonyl-sydnonimin und 10 g Troxerutin unter Rühren aufgelöst und das Volumen der Lösung durch Zusatz von Wasser für Injektionszwecke auf 5 Liter eingestellt.

Beispiel 3

Eine erfindungsgemässe, lichtstabilisierte Lösung von 3-(4-Methylsulfonylpiperazin-1-yl)-N-ethoxycarbonylsydnonimin für Ampullen bzw. Infusionsampullen hat beispielsweise folgende Zusammensetzung:

| | |
|---|---|
| 3-(4-Methylsulfonylpiperazin-1-yl)-N-ethoxycarbonylsydnonimin | 2 mg |
| Troxerutin | 100 mg |
| Wasser für Injektionszwecke ad | 1 ml |

Herstellung

Die Herstellung erfolgt wie im Beispiel 2 beschrieben, mit dem Unterschied, dass anstelle der dort angegebenen Menge 500 g Troxerutin eingesetzt werden.

Beispiel 4

Eine erfindungsgemässe, lichtstabilisierte Lösung von 3-(4-Methylsulfonylpiperazin-1-yl)-N-ethoxycarbonylsydnonimin für Ampullen bzw. Infusionsampullen hat beispielsweise folgende Zusammensetzung:

| | |
|---|---|
| 3-(4-Methylsulfonylpiperazin-1-yl)-N-ethoxycarbonylsydnonimin | 2 mg |
| Troxerutin | 40 mg |
| Wasser für Injektionszwecke ad | 1 ml |

Herstellung

Die Herstellung erfolgt wie im Beispiel 2 beschrieben, mit dem Unterschied, dass anstelle der dort angegebenen Menge 200 g Troxerutin eingesetzt werden.

Beispiel 5

Eine erfindungsgemässe, lichtstabilisierte Molsidomintablette hat beispielsweise folgende Zusammensetzung:

| | |
|---|---|
| Molsidomin | 2 mg |
| Apigetrin | 2 mg |
| Maisstärke | 40 mg |
| Milchzucker | 115 mg |
| Magnesiumstearat | 1 mg |

Apigetrin:

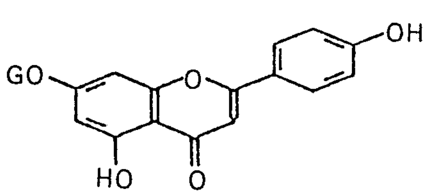

G=Glucose

Herstellung:

20 g Molsidomin, 20 g Apigetrin, 400 g Maisstärke, 1150 g Milchzucker und 10 g Magnesiumstearat werden in einem Mischaggregat homogen gemischt, und dann wird die Mischung zu Tabletten von 160 mg Gewicht verpresst.

Beispiel 6

Molsidomin-Retardtabletten ohne und mit wechselnden Mengen Photostabilisator Luteolin werden in folgender Zusammensetzung hergestellt:

| | | |
|---|---|---|
| a) | Molsidomin | 8 mg |
| | Mikrokristalline Zellulose | 40 mg |
| | Ricinusöl, hydriert | 30 mg |
| | Milchzucker | 120 mg |
| | Magnesiumstearat | 2 mg |

b), c), d)

| | |
|---|---|
| Molsidomin | 8 mg |
| Luteolin b) 8 mg, c) 0,8 mg; d) 0,08 mg | |
| Mikrokristalline Zellulose | 40 mg |
| Ricinusöl, hydriert | 30 mg |
| Milchzucker b) 112 mg; c) 119,2 mg; d) 120 mg | |
| Magnesiumstearat | 2 mg |

Herstellung der Zubereitung a)

80 g Molsidomin, 400 g mikrokristalline Zellulose, 300 g Ricinusöl, hydriert, und 1200 g Milchzucker werden in einem schnellaufenden Mischer gemischt, bis sich aufgrund der Friktionswärme ein Granulat bildet. Nach Abkühlen auf Zimmertemperatur wird das Granulat über ein 1,2 mm-Sieb passiert und nach Zumischen von 20 g Magnesiumstearat zu Tabletten von 200 mg Gewicht verpresst:

Herstellung der Zubereitungen b), c) und d):

80 g Molsidomin, b) 80 g, c) 8 g, d) 0,8 g Luteolin, 400 g mikrokristalline Zellulose, 300 g Ricinusöl, hydriert, und b) 1120 g, c) 1192 g, d) 1200 g Milchzucker werden in einem schnellaufenden Mischer gemischt, bis sich aufgrund der Friktionswärme ein Granulat bildet. Nach Abkühlen auf Zimmertemperatur wird das Granulat über ein 1,2 mm-Sieb passiert und nach Zumischen von 20 g Magnesiumstearat zu Tabletten von 200 mg Gewicht verpresst.

Die Tabletten der Zusammensetzung a), b), c) und d) werden der Belichtung in einem Suntest-Gerät der Firme Heraeus, Hanau, unterzogen und der Molsidominabbau gemessen. Die erhaltenen Molsidominmengen pro Tablette nach verschiedenen Belichtungszeiten sind der Fig. 3 zu entnehmen. Für einen 50%igen Molsidominabbau ergeben sich bei den verschiedenen Rezepturen die folgenden Belichtungszeiten:

a) 4,4 Std. b) 31,0 Std. c) 15,5 Std. d) 9,5 Std.

Man erkennt, dass in dieser festen Zubereitung eine Stabilisatorkonzentration von nur 1/100 der Wirkstoffkonzentration noch eine erhebliche Photostabilisierung des Molsidomins bewirkt.

Beispiel 7

Molsidomin-Tropfen ohne und mit wechselnden Mengen Photostabilisator Kämpferol-7,4'-dimethylether werden in folgender Zusammensetzung hergestellt:

| | | |
|---|---|---|
| a) | Molsidomin | 1,8 mg |
| | Methylparaben | 0,7 mg |
| | Propylparaben | 0,3 mg |
| | entmineralisiertes Wasser/Ethanol (1:1) ad | 1 ml |
| b), c), d), e), | Molsidomin | 1,8 mg |
| | Kämpferol-7,4'-dimethylether b) 18 mg, c) 1,8 mg, d) 0,18 mg, e) 0,0018 mg | |
| | Methylparaben | 0,7 mg |
| | Propylparaben | 0,3 mg |
| | entmineralisiertes Wasser/Ethanol (1:1) ad | 1 ml |

Kämpferol-7,4'-dimethylether:

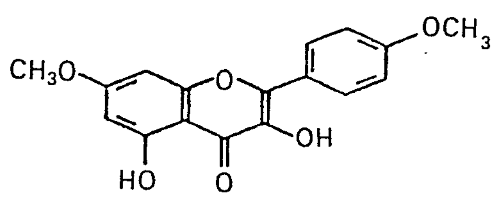

Herstellung der Zubereitung a)

In 4 Liter entmineralisiertem Wasser/Ethanol (1:1) werden 3,5 g Methylparaben und 1,5 g Propylparaben gelöst; anschliessend werden in dieser Lösung 9 g Molsidomin unter Rühren aufgelöst und das Volumen der Lösung durch Zusatz von entmineralisiertem Wasser/Ethanol (1:1) auf 5 Liter eingestellt.

Herstellung der Zubereitungen b), c), d), e)

In 4 Liter entmineralisiertem Wasser/Ethanol (1:1) werden 3,5 g Methylparaben und 1,5 g Propylparaben gelöst; anschliessend werden in dieser Lösung 9 g Molsidomin und b) 90 g, c) 9 g, d) 0,9 g,e) 0,009 g Kämpferol-7,4'-dimethylether unter Rühren aufgelöst und das Volumen der Lösung durch Zusatz von entmineralisiertem Wasser/Ethanol (1:1) auf 5 Liter eingestellt.

Die Tropfen der Zusammensetzung a) bis e) werden abgefüllt in Braunglasflaschen und 15 Stunden in einem Suntest-Gerät der Firma Heraeus, Hanau, belichtet und der Molsidominabbau gemessen. Folgende Molsidominkonzentrationen wurden bei den verschiedenen Rezepturen gemessen: (Anfangskonzentration = 100%)

a) 20% b) >99% c) 96% d) 87% e) 73%

Man erkennt, dass in dieser flüssigen Zubereitung eine Stabilisierungskonzentration von nur 1/1000 der Wirkstoffkonzentration noch eine erhebliche Photostabilisierung des Molsidomins bewirkt.

Beispiel 8

3-(S-Dioxo-tetrahydrothien-3-yl-methyl-amino)- N-acetylsydnonimin-Tropfen ohne und mit wechselnden Mengen verschiedener Photostabilisatoren werden in folgender Zusammensetzung hergestellt:

Stabilisatorfreie Zubereitung:

| | |
|---|---|
| 3-(S-Dioxo-tetrahydrothien-3-yl)-methyl-amino)-N-acetylsydnonimin | 1,8 mg |
| Methylparaben | 0,7 mg |
| Propylparaben | 0,3 mg |
| entmineralisiertes Wasser ad | 1 ml |

Stabilisatorhaltige Zubereitungen:

| | |
|---|---|
| 3-(S-Dioxo-tetrahydrothien-3-yl-methyl-amino)-N-acetyl-sydnonimin | 1,8 mg |
| Erfindungsgemässer Photostabilitor | laut Tabelle Spalten A+B, |
| Methylparaben | 0,7 mg |
| Propylparaben | 0,3 mg |
| entmineralisiertes Wasser ad | 1 ml |

Herstellung

In 3 Liter entmineralisiertem Wasser werden bei Siedetemperatur 3,5 g Methylparaben und 1,5 g Propylparaben gelöst. Nach Abkühlen auf Zimmertemperatur werden in dieser Lösung 9 g 3-(S-Dioxo-tetrahydrothien-3-yl-methyl-amino)- N-acetyl-sydnonimin und der erfindungsgemässe Photostabilisator laut Tabelle 1, Spalte A+C unter Rühren aufgelöst und das Volumen der Lösung durch Zusatz von entmineralisiertem Wasser auf 5 Liter eingestellt. Die so hergestellten Tropfen werden, abgefüllt in Braunglasflaschen, der Belichtung in einem Suntest-Gerät der Fa. Heraeus, Hanau, unterzogen, und der Sydnoniminabbau wird gemessen. Die hieraus errechneten Regressionskoeffizienten bei den verschiedenen Rezepturen sind der Spalte D der folgenden Tabelle 1 zu entnehmen.

Tabelle 1

| A Erfindungsgemässer Photostabilisator | B Menge (mg) pro 1 ml d. Zubereitung | C Eingesetzte Menge (g) bei der Herstellung | D Regressionskoeffizient |
|---|---|---|---|
| Ohne Stabilisator | 0 | 0 | -5,19 |
| Mono-Rutin-schwefelsäureester (Na-Salz) | 1,8 | 9 | -0,29 |
| do. | 0,18 | 0,9 | -1,9 |
| Mono-Hesperidin-phosphorsäureester (Na-Salz) | 1,8 | 9 | -1,80 |
| do. | 0,18 | 0,9 | -2,6 |

Fortsetzung Tabelle 1

| A Erfindungsgemässer Photostabilisator | B Menge (mg) pro 1 ml d. Zubereitung | C Eingesetzte Menge (g) bei der Herstellung | D Regressionskoeffizient |
|---|---|---|---|
| Hesperidin-methylchalcon | 1,8 | 9 | −2,2 |

Beispiel 9

In 100 ml 0,1 m Phosphatpuffer pH 7 werden 2 mg (8,26 μ Mol) Molsidomin gelöst. Danach wird die Lösung halbiert. In der einen Hälfte der Molsidominlösung werden 3,00 mg (4,13 μ Mol) Troxerutin gelöst. Proben beider Lösungen werden, abgefüllt in braune Probengläser, im Suntest-Gerät belichtet, die Abnahme der Molsidominkonzentration gemessen und die Regressionskoeffizienten berechnet.

Man erhält für die troxerutinfreie Probe den Wert 1,91, für die troxerutinhaltige Probe den Wert 0,67, was einem Lichtschutzfaktor von 2,85 entspricht.

Werden anstelle von Molsidomin in obigem Beispiel jeweils 8,26 μ Mol der aus Tabelle ersichtlichen Sydnonimine eingesetzt, so erhält man die angegebenen Regressions- und Schutzwerte.

Tabelle 2

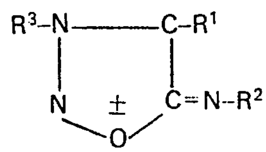

| $R^3$ | $R^2$ | $R^1$ | MG | Regressionskoeffizient ohne Troxerutin | Regressionskoeffizient mit Troxerutin | Schutzfaktor |
|---|---|---|---|---|---|---|
| 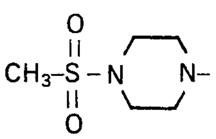 | 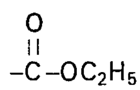 | H | 319 | −2,67 | −0,75 | 3,56 |
| 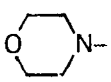 | 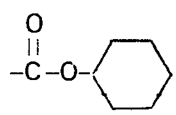 | H | 280 | −2,93 | −0,86 | 3,41 |
| 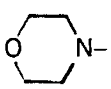 | 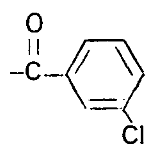 | H | 308,7 | −2,93 | −1,23 | 2,38 |
| 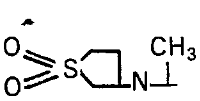 | 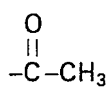 | H | 310,8 | −1,99 | −0,73 | 2,73 |
| 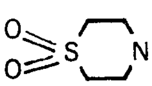 | 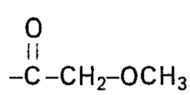 | H | 290 | −3,23 | −0,99 | 3,26 |
| 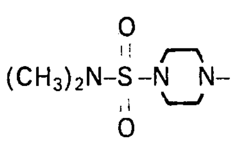 | 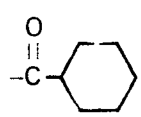 | H | 386,5 | −3,10 | −0,93 | 3,33 |
| 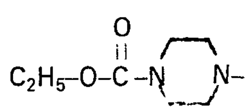 | 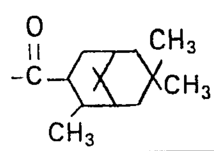 | H | 405,5 | −3,20 | −1,00 | 3,20 |

Bei den folgenden Produkten wurde durch farbloses Glas belichtet und als Lösungsmittel n/100 Salzsäure benutzt.

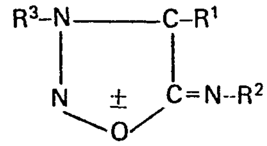

| $R^3$ | $R^2$ | $R^1$ | MG | Regressions-koeffizient ohne Troxerutin | Regressions-koeffizient mit Troxerutin | Schutz-faktor |
|---|---|---|---|---|---|---|
| 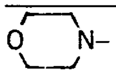 | –H | H | 206,5 | –2,90 | –0,63 | 4,63 |
| 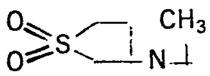 | –H | H | 236,7 | –5,73 | –1,67 | 3,44 |

Beispiel 10

In 500 ml eines 0,1 molaren Phosphatpuffers vom pH 7,0 werden 8,26 μ Mol Molsidomin, aufgelöst. Zu je 50 ml dieser Lösung werden dann je 8,26 μ Mol der in der folgenden Tabelle 3 angegebenen, erfindungsgemässen Photostabilisatoren, gelöst in 5 ml (Lösungsmodus A) oder in 25 ml (Lösungsmodus B) oder in 50 ml Ethanol (Lösungsmodus C), zugesetzt und das Volumen der Mischung durch Zusatz des Phosphatpuffers auf 100 ml eingestellt.

Die Lösungen werden unter einem Braunfilter im «Suntest»-Gerät belichtet, die Molsidominkonzentration nach verschiedenen Belichtungszeiten bestimmt und der Regressionskoeffizient und der Schutzfaktor errechnet. Folgende Werte wurden erhalten:

Ohne erfindungsgemässen Photostabilisator ergibt sich für die Molsidominlösung ein Regressionskoeffizient von –1,66.

Tabelle 3

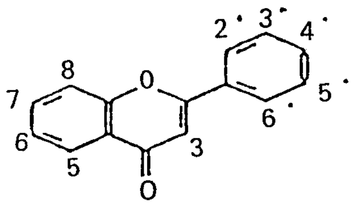

| 3 | 5 | 6 | 7 | 8 | 2′ | 3′ | 4′ | 5′ | 6′ | G | X | Lösungs-modus | Regress.-koeffi-zient | Schutz-faktor |
|---|---|---|---|---|---|---|---|---|---|---|---|---|---|---|
| OH | OH | | OH | | | | | | | | | A | −0,67 | 2,5 |
| OH | OH | | OH | | | | OH | | | | | A | −0,55 | 3,0 |
| OH | OH | | OG | | | | OH | | | Neohespe-ridose | | A | −0,60 | 2,8 |
| $OCH_3$ | OH | | OH | | | | $OCH_3$ | | | | | C | −0,71 | 2,3 |
| OH | OH | | $OCH_3$ | | | | $OCH_3$ | | | | | C | −0,36 | 4,6 |
| $OCH_3$ | OH | | $OCH_3$ | | | | $OCH_3$ | | | | | C | −0,83 | 2,0 |
| OH | OH | | $OCH_3$ | X | | | $OCH_3$ | | | | Butyryloxy | C | −0,89 | 1,9 |
| OH | OH | | OH | | | | $OCH_3$ | | | | | A | −0,57 | 2,9 |
| OH | OH | | $OCH_3$ | | | $OCH_3$ | OH | | | | | C | −0,83 | 2,0 |
| OH | OH | | OH | | OH | | OH | | | | | A | −0,55 | 3,0 |
| OG | OH | | OH | | | OH | OH | | | Arabinose | | A | −0,53 | 3,2,5 |
| OG | OH | | OH | | | OH | OH | | | Glucose | | A | −0,60 | 2,8 |
| OG | OH | | OH | | | OH | OH | | | Galactose | | A | −0,61 | 2,7 |
| OG | OH | | OH | | | OH | OH | OH | | Rhamnose | | C | −0,83 | 2,0 |
| OH | OH | | OH | | | OH | OH | OH | | | | C | −0,58 | 2,9 |
| OG | OH | | X | | | X | X | | | Rutinose | | A | −0,45 | 3,7 |
| $OCH_3$ | OH | | $OCH_3$ | | | OH | OH | | | | | B | −0,61 | 2,7 |
| $OCH_3$ | OH | | $OCH_3$ | | | $OCH_3$ | $OCH_3$ | | | | | C | −0,69 | 2,4 |
| | OH | | OH | | | | OH | | | | | C | −0,70 | 2,4 |
| | OH | | OG | | | | OH | | | Glucose | | A | −0,57 | 3,0 |
| OH | | | OH | | | OH | OH | | | | | C | −0,80 | 2,1,5 |
| OH | OH | | OH | | | OH | OH | | | | | A | −0,52 | 3,2 |
| OG | OH | | OH | | | OH | OH | | | Rutinose | | A | −0,47 | 3,6 |
| OG | OH | | OH | | | OH | OH | | | Rhamnose | | A | −0,52 | 3,2 |
| | OH | | $OCH_3$ | | | | $OCH_3$ | | | | | C | −0,57 | 2,9 |
| | OH | | OH | | | OH | OH | | | | | A | −0,47 | 3,5 |

Fortsetzung Tabelle 3

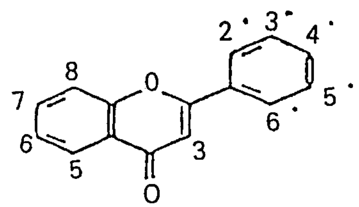

| 3 | 5 | 6 | 7 | 8 | 2' | 3' | 4' | 5' | 6' | G | X | Lösungs-modus | Regress.-koeffi-zient | Schutz-faktor |
|---|---|---|---|---|---|---|---|---|---|---|---|---|---|---|
| | OH | | OG | | | OH | OH | | | Glucose | | C | −0,51 | 3,2 |
| | OH | | OH | | | OH | $OCH_3$ | | | | | C | −0,62 | 2,7 |
| | OH | | OH | | | | | | | | | C | −0,83 | 2,0 |

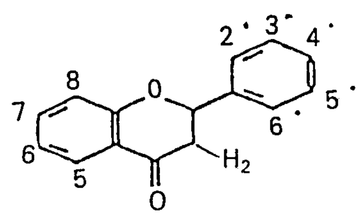

| 3 | 5 | 6 | 7 | 8 | 2' | 3' | 4' | 5' | 6' | G | X | Lösungs-modus | Regress.-koeffi-zient | Schutz-faktor |
|---|---|---|---|---|---|---|---|---|---|---|---|---|---|---|
| | OH | | OG | | | | OH | | | Neohespe-ridose | | B | −1,0 | 1,6 |
| | OH | | OH | | | | OH | | | | | A | −0,53 | 3,2 |
| | OH | | OH | | | OH | OH | | | | | A | −0,58 | 2,9 |
| | OH | | OH | | | OH | $OCH_3$ | | | | | A | −0,58 | 2,9 |
| | OH | X | OH | | | | | | | | $-CH_3$ | A | −0,77 | 2,2 |
| | OH | | OH | X | | | | | | | $-CH_3$ | A | −0,62 | 2,7 |

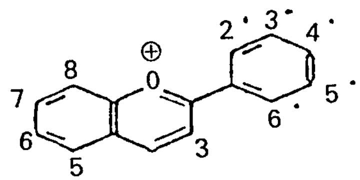

| | | | | | | | |
|---|---|---|---|---|---|---|---|
| OG | OG | OH | OH | OH | B | −0,82 | 2,0 |

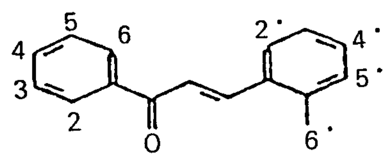

| 2 | 3 | 4 | 5 | 6 | 2′ | 3′ | 4′ | 5′ | 6′ | G | X | Lösungs-modus | Regress.-koeffi-zient | Schutz-faktor |
|---|---|---|---|---|---|---|---|---|---|---|---|---|---|---|
| OH | | OH | | OH | | | OH | | | | | A | −0,80 | 2,1 |
| OH | | OH | | OH | | | OH | | | Glucose | | A | −0,62 | 2,7 |
| OG | | OG | | OH | | OH | $OCH_3$ | | | Rutinose | | A | −0,64 | 2,6 |

**Patentansprüche für die Vertragsstaaten BE, CH, DE, FR, GB, IT, LI, NL**

1. Photostabile Mischung eines Sydnonimins der allgemeinen Formel I

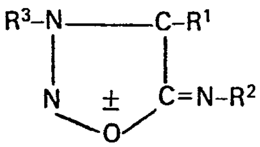

(I)

worin

$R^1$ Wasserstoff, Alkyl mit 1 bis 5 C-Atomen oder Halogen,

$R^2$ Wasserstoff, –NO, –COR$^4$ oder –SO$_2$R$^5$,

$R^3$ einen der Reste

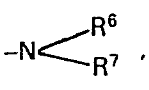 , 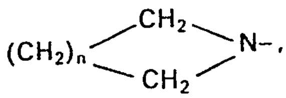 , 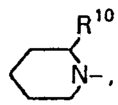 ,

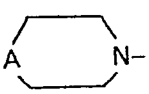 oder 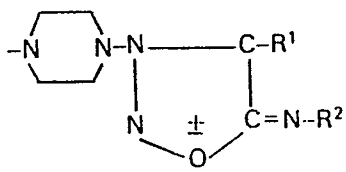

bedeuten, wobei

A für Sauerstoff oder eine der Gruppen >S(O)$_m$, >N–R$^{11}$, >N–COOR$^8$ oder >N–SO$_2$R$^9$ und

m für 0, 1 oder 2 und

n für 2, 3, 4, oder 5 stehen,

$R^4$ Wasserstoff, einen aliphatischen Rest mit 1 bis 6 C-Atomen, der auch durch Alkoxy mit 1 bis 6 C-Atomen oder Aryloxy mit 6 bis 12 C-Atomen substituiert sein kann, einen cycloaliphatischen Rest mit 5 bis 8 C-Atomen, einen bicycloaliphatischen Rest mit 7 bis 14 C-Atomen, einen tricycloaliphatischen Rest mit 7 bis 16 C-Atomen, Aryl mit 6 bis 12-Atomen, das auch durch Halogen und/oder Alkyl mit 1 bis 4 C-Atomen und/oder Alkoxi mit 1 bis 4 C-Atomen mono-, di- oder trisubstituiert sein kann, Aralkyl oder Aralkenyl mit 7 bis 13 C-Atomen, Alkoxy mit 1 bis 6 C-Atomen, Cycloalkoxy mit 5 bis 8 C-Atomen, Bicycloalkoxy mit 7 bis 12 C-Atomen, Tricycloalkoxy mit 7 bis 16 C-Atomen, Aryloxy mit 6 bis 12 C-Atomen, Pyridyl, Pyrrolyl, Imidazolyl, Pyrazolyl, Pyrazinyl, Pyrimidinyl, Pyridazinyl, Alkoxicarbonyl mit insgesamt 2 bis 7 C-Atomen,

$R^5$ Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem der Alkylreste,

$R^6$ und $R^7$ unabhängig voneinander Alkyl mit 1 bis 6 C-Atomen, Alkenyl mit 3 bis 6 C-Atomen, Cycloalkyl mit 5 bis 8 C-Atomen, Bicycloalkyl mit 7 bis 14 C-Atomen, Tricycloalkyl mit 7 bis 16 C-Atomen, Aralkyl mit 7 bis 9 C-Atomen, wobei die Aralkylgruppe durch Methyl oder Halogen substituiert sein kann, oder einen über eine Alkylenkette an das Stickstoffatom der 3-Aminogruppe gebundenen heterocyclischen Ring bedeuten und

$R^6$ zusätzlich Wasserstoff und

$R^7$ zusätzlich ein Rest der Formel 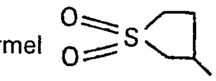 sein kann,

$R^8$ Alkyl mit 1 bis 4 C-Atomen,

$R^9$ Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem der Alkylreste,

$R^{10}$ Alkyl mit 1 bis 4 C-Atomen und

$R^{11}$ Alkyl mit 1 bis 6 C-Atomen bedeuten,

oder eines Salzes des Sydnonimins, einem Photostabilisator der allgemeinen Formel II

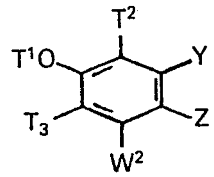

(II)

worin

$T^1$ Wasserstoff, Alkyl mit 1 oder 2 C-Atomen, β-Hydroxyethyl, oder einen Rest G,

$T^2$ Wasserstoff, Alkyl mit 1 oder 2 C-Atomen oder Alkanoyloxy mit 1 bis 4 C-Atomen,

$T^3$ Wasserstoff oder Alkyl mit 1 oder 2 C-Atomen,

Y eine Hydroxygruppe, Alkoxy mit 1 oder 2 C-Atomen oder β-Hydroxyethoxy und

Z eine Gruppe der Formel

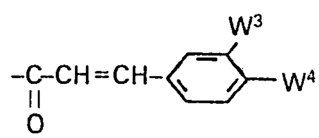

oder

Y und Z gemeinsam ein Strukturelement der Formeln III, IV oder V bedeuten

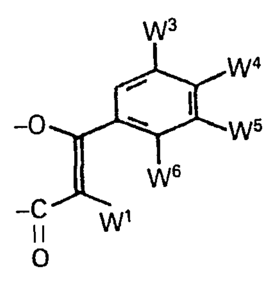

(III)

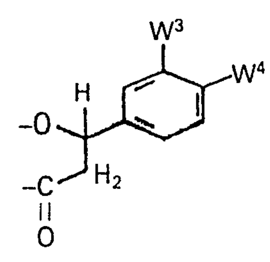

(IV)

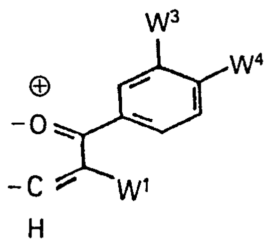

(V)

dessen freie Sauerstoffvalenz in der Y-Position und dessen freie Kohlenstoffvalenz in der Z-Position gebunden ist und

$W^1$ Wasserstoff oder eine der Gruppen –OH, Alkoxy mit 1 oder 2 C-Atomen oder –OG,

$W^2$ Wasserstoff oder eine der Gruppen –OH oder –OG,

$W^3$ Wasserstoff oder eine der Gruppen –OH, Alkoxy mit 1 oder 2 C-Atomen oder β-Hydroxyethoxy,

$W^4$ Wasserstoff oder eine der Gruppen –OH, Alkoxy mit 1 oder 2 C-Atomen oder β-Hydroxyethoxy,

$W^5$ Wasserstoff oder –OH,

$W^6$ Wasserstoff oder –OH und

G der Rest eines Mono- oder Disaccharids ist, und/oder einen Ester dieser Verbindungen mit Säuren sowie gegebenenfalls dessen Salz mit Basen und gegebenenfalls weitere Hilfs- und Zusatzstoffe mit Ausnahme einer Mischung von Molsidomin und einem oder mehreren Photostabilisatoren der allgemeinen Formel VI

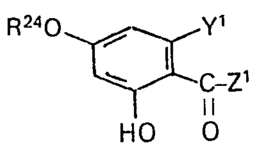

(VI)

worin

$Y^1$ eine Hydroxygruppe, Alkoxy mit 1 oder 2 C-Atomen oder β-Hydroxyethoxy und

$Z^1$ eine Gruppe der Formel

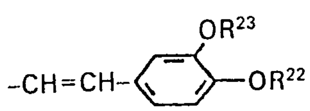

oder $Y^1$ und $Z^1$ gemeinsam ein Strukturelement der Formel VII oder VIII bedeuten

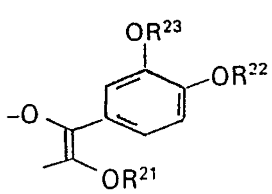

(VII)

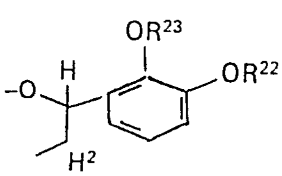

(VIII)

dessen freie Sauerstoffvalenz in der $Y^1$-Position und dessen freie Kohlenstoffvalenz in der $Z^1$-Position gebunden ist, und

$R^{21}$ Wasserstoff oder einen Rest $G^1$,

$R^{22}$ Wasserstoff, Alkyl mit 1 oder 2 C-Atomen oder β-Hydroxyethyl,

$R^{23}$ Wasserstoff, Alkyl mit 1 oder 2 C-Atomen oder β-Hydroxyethyl,

$R^{24}$ Wasserstoff, Alkyl mit 1 oder 2 C-Atomen, β-Hydroxyethyl oder einen Rest $G^1$ bedeuten und

$G^1$ der Rest eines Mono- oder Disaccharids ist, oder einem Ester der Verbindung der Formel VI mit einer pharmakologisch unbedenklichen Säure sowie gegebenenfalls dessen Salz mit einer pharmakologisch unbedenklichen Base.

2. Photostabile Mischung gemäss Anspruch 1, dadurch gekennzeichnet, dass das Sydnonimin der allgemeinen Formel Ia entspricht

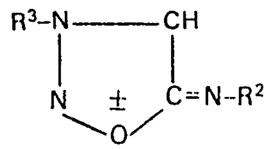

(Ia)

worin

$R^2$ Wasserstoff oder $-COR^4$,

$R^3$ einen der Reste

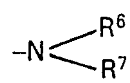

oder

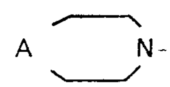

bedeuten, wobei

A für Sauerstoff oder eine der Gruppen $>S(O)_m$, $>N-COOR^8$ oder $>N-SO_2R^9$ und

m für 0, 1 oder 2 stehen,

$R^4$ Wasserstoff, Alkyl mit 1 bis 4 C-Atomen, das auch durch Alkoxy mit 1 bis 4 C-Atomen substituiert sein kann, Cycloalkyl mit 5 bis 8 C-Atomen, Bicycloalkyl mit 7 bis 12 C-Atomen, Phenyl, das auch durch Halogen substituiert sein kann, Alkoxy mit 1 bis 4 C-Atomen, Cycloalkoxy mit 5 bis 8 C-Atomen,

$R^6$ Wasserstoff oder Alkyl mit 1 bis 4 C-Atomen,

$R^7$ einen Rest der Formel

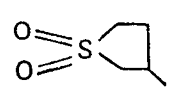

$R^8$ Alkyl mit 1 bis 4 C-Atomen und

$R^9$ Alkyl mit 1 bis 4 C-Atomen oder eine Dialkylaminogruppe mit 1 bis 4 C-Atomen in jedem der Alkylreste bedeuten.

3. Photostabilisierte pharmazeutische Zubereitung bestehend aus einem Wirkstoff der allgemeinen Formel I

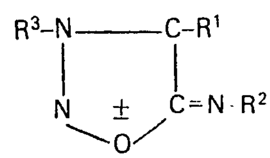

(I)

worin $R^1$, $R^2$ und $R^3$ die im Anspruch 1 angegebenen Bedeutungen haben, oder einem pharmakologisch unbedenklichen Salz des Wirkstoffs, einem Photostabilisator der im Anspruch 1 angegebenen Formel II oder einem Ester dieses Photostabilisators mit pharmakologisch unbedenklichen Säuren sowie gegebenenfalls dessen Salz mit pharmakologisch unbedenklichen Basen sowie gegebenenfalls weiteren in galenischen Zubereitungen üblichen Hilfs- und Zusatzstoffen.

4. Photostabilisierte pharmazeutische Zubereitung gemäss Anspruch 3 bestehend aus einem Wirkstoff der allgemeinen Formel Ia

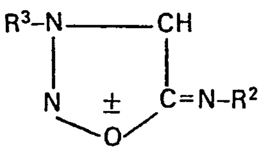

(Ia)

worin $R^2$ und $R^3$ die im Anspruch 2 angegebenen Bedeutungen haben, oder einem pharmakologisch unbedenklichen Salz des Wirkstoffs, einem Photostabilisator der im Anspruch 2 angegebenen Formel II oder einem Ester dieses Photostabilisators mit pharmakologisch unbedenklichen Säuren sowie gegebenenfalls dessen Salz mit pharmakologisch unbedenklichen Basen sowie gegebenenfalls weiteren in galenischen Zubereitungen üblichen Hilfs- und Zusatzstoffen.

5. Photostabilisierte Mischung bzw. pharmazeutische Zubereitung eines Sydnonimins gemäss den Ansprüchen 1 bis 4, dadurch gekennzeichnet, dass der Photostabilisator eine Verbindung der Formel II ist, worin

Y, Z, und soweit vorhanden, $W^1$ und G die im Anspruch 1 genannten Bedeutungen haben,

$W^3$ Hydroxy oder β-Hydroxyethoxy und

$W^4$ eine der Gruppen –OH, Alkoxy mit 1 oder 2 C-Atomen oder β-Hydroxyethoxy,

$T^1$ Wasserstoff, β-Hydroxyethyl oder G und

$T^2$ und $T^3$ Wasserstoff bedeuten, oder ein Ester dieser Verbindungen sowie gegebenenfalls dessen Salz ist.

6. Photostabilisierte Mischung bzw. pharmazeutische Zubereitung eines Sydnonimins gemäss den Ansprüchen 1 bis 4, dadurch gekennzeichnet, dass der Photostabilisator Hesperidin, Hesperetin, Quercetin und seine Derivate, Rutin, Naringenin, Kämpferol und seine Derivate, insbesondere sein 7,4'-Dimethylether, Morin, Apigetrin, Luteolin und dessen 7-Glucosid oder Troxerutin oder ein Ester dieser Verbindungen sowie gegebenenfalls dessen Salz ist.

7. Photostabilisierte Mischung bzw. pharmazeutische Zubereitung eines Sydnonimins gemäss den Ansprüchen 1 bis 4, dadurch gekennzeichnet, dass darin Molsidomin und der Photostabilisator im Gewichtsverhältnis von 1:0,001 bis 1:50 vorliegen.

8. Verfahren zur Photostabilisierung von Sydnoniminen der allgemeinen Formel I

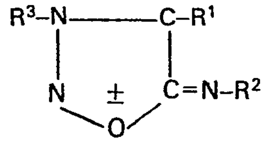

(I)

worin

$R^1$, $R^2$ und $R^3$ die im Anspruch 1 angegebenen Bedeutungen haben, dadurch gekennzeichnet, dass man es mit einem Photostabilisator der allgemeinen Formel II

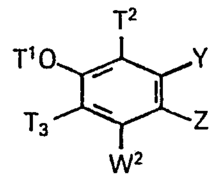

(II)

worin $T^1$ bis $T^3$, $W^2$, Y und Z die im Anspruch 1 angegebenen Bedeutungen haben, und/oder einem Ester dieser Verbindungen mit Säuren sowie gegebenenfalls dessen Salz mit Basen und gegebenenfalls weiteren Hilfs- und Zusatzstoffen gründlich vermischt und homogenisiert.

9. Verwendung von Verbindungen der allgemeinen Formel II

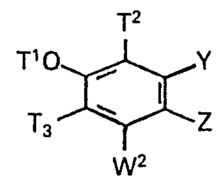

(II)

worin $T^1$ bis $T^3$, $W^2$, Y und Z die im Anspruch 1 angegebenen Bedeutungen haben, und/oder einem Ester dieser Verbindungen mit Säuren sowie gegebenenfalls dessen Salz nur Photostabilisierung von Sydnoniminen der allgemeinen Formel I

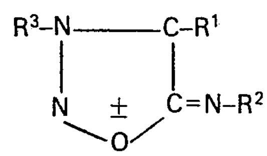

(I)

worin

$R^1$, $R^2$ und $R^3$ die im Anspruch 1 angegebenen Bedeutungen haben, und deren Salzen.

10. Verwendung gemäss Anspruch 9 von Verbindungen der Formel II und deren Estern mit pharmakologisch unbedenklichen Säuren sowie gegebenenfalls deren Salzen mit pharmakologisch unbedenklichen Basen zur Photostabilisierung von Sydnoniminen der Formeln I bzw. Ia und deren pharmakologisch unbedenklichen Salzen.

**Patentansprüche für den Vertragsstaat: AT**

1. Verfahren zur Photostabilisierung eines Sydnonimins der allgemeinen Formel I

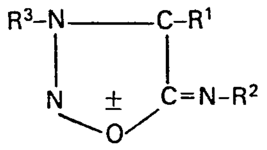

(I)

worin

$R^1$ Wasserstoff, Alkyl mit 1 bis 5 C-Atomen oder Halogen,

$R^2$ Wasserstoff, –NO, $-COR^4$ oder $-SO_2R^5$,

$R^3$ einen der Reste

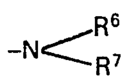 , 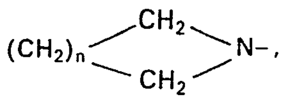 ,

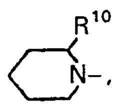 , 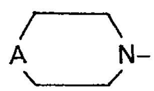 oder

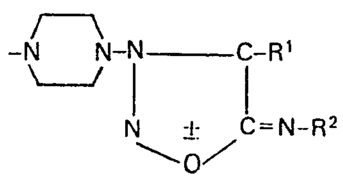

bedeuten, wobei

A für Sauerstoff oder eine der Gruppen $>S(O)_m$, $>N-R^{11}$, $>N-COOR^8$ oder $>N-SO_2R^9$

und

m für 0, 1 oder 2 und

n für 2, 3, 4, oder 5 stehen,

$R^4$ Wasserstoff, einen aliphatischen Rest mit 1 bis 6 C-Atomen, der auch durch Alkoxy mit 1 bis 6 C-Atomen oder Aryloxy mit 6 bis 12 C-Atomen substituiert sein kann, einen cycloaliphatischen Rest mit 5 bis 8 C-Atomen, einen bicycloaliphatischen Rest mit 7 bis 14 C-Atomen, einen tricycloaliphatischen Rest mit 7 bis 16 C-Atomen, Aryl mit 6 bis 12-Atomen, das auch durch Halogen und/oder Alkyl mit 1 bis 4 C-Atomen und/oder Alkoxi mit 1 bis 4 C-Atomen mono-, di- oder trisubstituiert sein kann, Aralkyl oder Aralkenyl mit 7 bis 13 C-Atomen, Alkoxy mit 1 bis 6 C-Atomen, Cycloalkoxy mit 5 bis 8 C-Atomen, Bicycloalkoxy mit 7 bis 12 C-Atomen, Tricycloalkoxy mit 7 bis 16 C-Atomen, Aryloxy mit 6 bis 12 C-Atomen, Pyridyl Pyrrolyl, Imidazolyl, Pyrazolyl, Pyrazinyl, Pyrimidinyl, Pyridazinyl, Alkoxicarbonyl mit insgesamt 2 bis 7 C-Atomen,

$R^5$ Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem der Alkylreste,

$R^6$ und $R^7$ unabhängig voneinander Alkyl mit 1 bis 6 C-Atomen, Alkenyl mit 3 bis 6 C-Atomen, Cycloalkyl mit 5 bis 8 C-Atomen, Bicycloalkyl mit 7 bis 14 C-Atomen, Tricycloalkyl mit 7 bis 16 C-Atomen, Aralkyl mit 7 bis 9 C-Atomen, wobei die Aralkylgruppe durch Methyl oder Halogen substituiert sein kann, oder einen über eine Alkylenkette an das Stickstoffatom der 3-Aminogruppe gebundenen heterocyclischen Ring bedeuten und

$R^6$ zusätzlich Wasserstoff und $R^7$ zusätzlich ein Rest der Formel

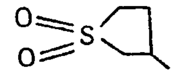

sein kann,

$R^8$ Alkyl mit 1 bis 4 C-Atomen,

$R^9$ Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem der Alkylreste,

$R^{10}$ Alkyl mit 1 bis 4 C-Atomen und

$R^{11}$ Alkyl mit 1 bis 6 C-Atomen bedeuten, oder eines Salzes des Sydnonimins, dadurch gekennzeichnet, dass man das Sydnonimin oder sein Salz mit einem Photostabilisator der allgemeinen Formel II

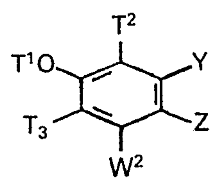

(II)

worin

$T^1$ Wasserstoff, Alkyl mit 1 oder 2 C-Atomen, β-Hydroxyethyl, oder einen Rest G,

$T^2$ Wasserstoff, Alkyl mit 1 oder 2 C-Atomen oder Alkanonyloxy mit 1 bis 4 C-Atomen,

$T^3$ Wasserstoff oder Alkyl mit 1 oder 2 C-Atomen,

Y eine Hydroxygruppe, Alkoxy mit 1 oder 2 C-Atomen oder β-Hydroxyethoxy und

Z eine Gruppe der Formel

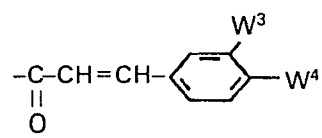

oder

Y und Z gemeinsam ein Strukturelement der Formeln III, IV oder V bedeuten

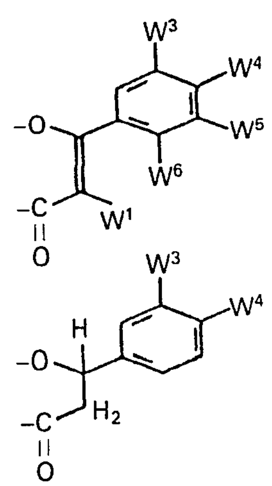

(III)

(IV)

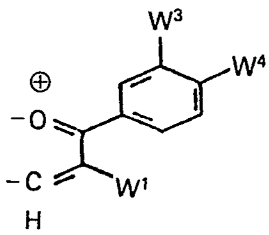

(V)

dessen freie Sauerstoffvalenz in der Y-Position und dessen freie Kohlenstoffvalenz in der Z-Position gebunden ist und

$W^1$ Wasserstoff oder eine der Gruppen –OH, Alkoxy mit 1 oder 2 C-Atomen oder –OG,

$W^2$ Wasserstoff oder eine der Gruppen –OH oder –OG,

$W^3$ Wasserstoff oder eine der Gruppen –OH, Alkoxy mit 1 oder 2 C-Atomen oder β-Hydroxyethoxy,

$W^4$ Wasserstoff oder eine der Gruppen –OH, Alkoxy mit 1 oder 2 C-Atomen oder β-Hydroxyethoxy,

$W^5$ Wasserstoff oder –OH,

$W^6$ Wasserstoff oder –OH und

G der Rest eines Mono- oder Disaccharids ist, und/oder einen Ester dieser Verbindungen mit Säuren sowie gegebenenfalls dessen Salz mit Basen und gegebenenfalls weitere Hilfs- und Zusatzstoffe mit Ausnahme eines Verfahrens zur Photostabilisierung von Molsidomin und von Mischungen von Molsidomin mit Mischungskomponenten, wobei man dem Molsidomin bzw. seinen Mischungen als Photostabilisator eine oder mehrere Verbindungen der allgemeinen Formel VI

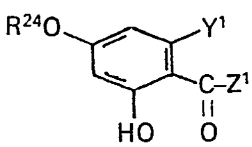

(VI)

worin

$Y^1$ eine Hydroxygruppe, Alkoxy mit 1 oder 2 C-Atomen oder β-Hydroxyethoxy und

$Z^1$ eine Gruppe der Formel

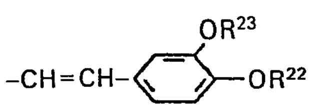

oder $Y^1$ und $Z^1$ gemeinsam ein Strukturelement der Formel VII oder VIII bedeuten

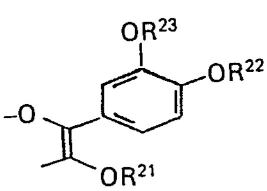

(VII)

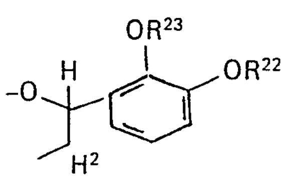

(VIII)

dessen freie Sauerstoffvalenz in der $Y^1$-Position und dessen freie Kohlenstoffvalenz in der $Z^1$-Position gebunden ist, und

$R^{21}$ Wasserstoff oder einen Rest $G^1$,

$R^{22}$ Wasserstoff, Alkyl mit 1 oder 2 C-Atomen oder β-Hydroxyethyl,

$R^{23}$ Wasserstoff, Alkyl mit 1 oder 2 C-Atomen oder β-Hydroxyethyl,

$R^{24}$ Wasserstoff, Alkyl mit 1 oder 2 C-Atomen, β-Hydroxyethyl oder einen Rest $G^1$ bedeuten und

$G^1$ der Rest eines Mono- oder Disaccharids ist, oder einem Ester der Verbindung der Formel VI mit einer pharmakologisch unbedenklichen Säure sowie gegebenenfalls dessen Salz mit einer pharmakologisch unbedenklichen Base.

2. Verfahren gemäss Anspruch 1, dadurch gekennzeichnet, dass das Sydnonimin der allgemeinen Formel Ia entspricht

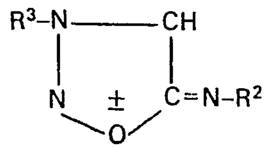

(Ia)

worin

$R^2$ Wasserstoff oder $-COR^4$,

$R^3$ einen der Reste

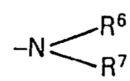

oder

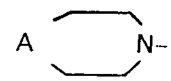

bedeuten, wobei

A für Sauerstoff oder eine der Gruppen $>S(O)_m$, $>N-COOR^8$ oder $>N-SO_2R^9$ und

m für 0, 1 oder 2 stehen,

$R^4$ Wasserstoff, Alkyl mit 1 bis 4 C-Atomen, das auch durch Alkoxy mit 1 bis 4 C-Atomen substituiert sein kann, Cycloalkyl mit 5 bis 8 C-Atomen, Bicycloalkyl mit 7 bis 12 C-Atomen, Phenyl, das auch durch Halogen substituiert sein kann, Alkoxy mit 1 bis 4 C-Atomen, Cycloalkoxy mit 5 bis 8 C-Atomen,

$R^6$ Wasserstoff oder Alkyl mit 1 bis 4 C-Atomen,

$R^7$ einen Rest der Formel

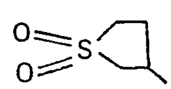

$R^8$ Alkyl mit 1 bis 4 C-Atomen und

$R^9$ Alkyl mit 1 bis 4 C-Atomen oder eine Dialkylaminogruppe mit 1 bis 4 C-Atomen in jedem der Alkylreste bedeuten.

3. Verfahren nach Anspruch 1, dadurch gekennzeichnet, dass man zur Herstellung photostabilisierter pharmazeutischer Zubereitungen einen Wirkstoff der allgemeinen Formel I

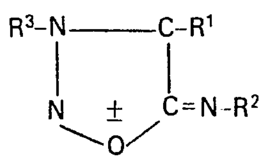

(I)

worin $R^1$, $R^2$ und $R^3$ die im Anspruch 1 angegebenen Bedeutungen haben, oder ein pharmakologisch unbedenkliches Salz des Wirkstoffs, einen Photostabilisator der im Anspruch 1 angegebenen Formel II oder einen Ester dieses Photostabilisators mit pharmakologisch unbedenklichen Säuren sowie gegebenenfalls dessen Salz mit pharmakologisch unbedenklichen Basen sowie gegebenenfalls weiteren in galenischen Zubereitungen üblichen Hilfs- und Zusatzstoffen verwendet.

4. Verfahren nach Anspruch 2, dadurch gekennzeichnet, dass man zur Herstellung photostabilisierter pharmazeutischer Zubereitungen einen Wirkstoff der allgemeinen Formel Ia

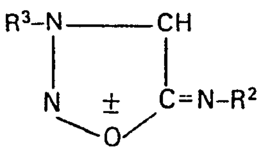

(Ia)

worin $R^2$ und $R^3$ die im Anspruch 2 angegebenen Bedeutungen haben, oder ein pharmakologisch unbedenkliches Salz des Wirkstoffs, einen Photostabilisator der im Anspruch 2 angegebenen Formel II oder einen Ester dieses Photostabilisators mit pharmakologisch unbedenklichen Säuren sowie gegebenenfalls dessen Salz mit pharmakologisch unbedenklichen Basen sowie gegebenenfalls weiteren in galenischen Zubereitungen üblichen Hilfs- und Zusatzstoffen verwendet.

5. Verfahren gemäss den Ansprüchen 1 bis 4, dadurch gekennzeichnet, dass man als Photostabilisator eine Verbindung der Formel II verwendet, worin

Y, Z, und soweit vorhanden, $W^1$ und G die im Anspruch 1 genannten Bedeutungen haben,

$W^3$ Hydroxy oder β-Hydroxyethoxy und

$W^4$ eine der Gruppen $-OH$, Alkoxy mit 1 oder 2 C-Atomen oder β-Hydroxyethoxy,

$T^1$ Wasserstoff, β-Hydroxyethyl oder G und

$T^2$ und $T^3$ Wasserstoff bedeuten, oder dass man einen Ester dieser Verbindungen sowie gegebenenfalls dessen Salz verwendet.

6. Verfahren gemäss den Ansprüchen 1 bis 4, dadurch gekennzeichnet, dass man als Photostabilisator Hesperidin, Hesperetin, Quercetin und seine Derivate, Rutin, Naringenin, Kämpferol und seine Derivate, insbesondere seinen 7,4'-Dimethylether, Morin, Apigetrin, Luteolin und dessen 7-Glucosid oder Troxerutin oder einen Ester dieser Verbindungen sowie gegebenenfalls dessen Salz verwendet.

7. Verfahren gemäss Ansprüchen 1 bis 4, dadurch gekennzeichnet, dass man Molsidomin und einen Photostabilisator im Gewichtsverhältnis von 1:0,001 bis 1:50 verwendet.

8. Verfahren gemäss den Ansprüchen 1 bis 7, dadurch gekennzeichnet, dass die Komponenten gründlich vermischt und homogenisiert werden.

9. Verwendung von Verbindungen der allgemeinen Formel II

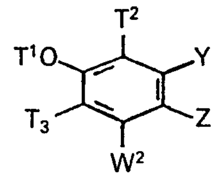

(II)

worin $T^1$ bis $T^3$, $W^2$, Y und Z die im Anspruch 1 angegebenen Bedeutungen haben, und/oder einem Ester dieser Verbindungen mit Säuren sowie gegebenenfalls dessen Salz zur Photostabilisierung von Sydnoniminen der allgemeinen Formel I

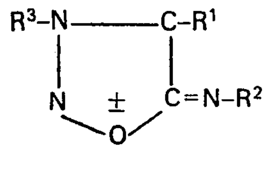

(I)

worin

$R^1$, $R^2$ und $R^3$ die im Anspruch 1 angegebenen Bedeutungen haben, und deren Salzen.

10. Verwendung gemäss Anspruch 9 von Verbindungen der Formel II und deren Estern mit pharmakologisch unbedenklichen Säuren sowie gegebenenfalls deren Salzen mit pharmakologisch unbedenklichen Basen zur Photostabilisierung von Sydnoniminen der Formeln I bzw. Ia und deren pharmakologisch unbedenklichen Salzen.

**Revendications pour les Etats contractants: BE, CH, DE, FR, GB, IT, LI, NL**

1. Mélange photostable constitué:

– d'une sydnonimine répondant à la formule générale I:

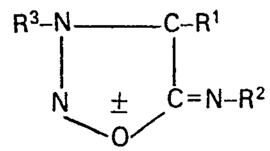

(I)

dans laquelle:

$R^1$ représente l'hydrogène, un alkyle contenant de 1 à 5 atomes de carbone ou un halogène,

$R^2$ représente l'hydrogène, $-NO$, $-COR^4$ ou $-SO_2R^5$,

$R^3$ représente l'un des radicaux suivants:

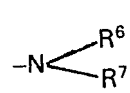 , 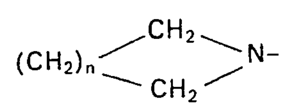 ,

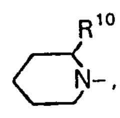 , 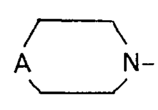 et

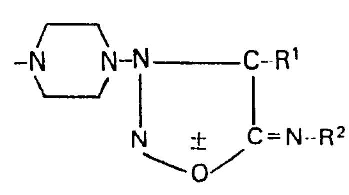

A représente l'oxygène ou un radical $>S(O)_m$, $>N-R^{11}$, $>N-COOR^8$ ou $>N-SO_2R^9$,

m est égal à 0, à 1 ou à 2,

n est égal à 2, à 3, à 4 ou à 5,

$R^4$ représente:

- l'hydrogène,
- un radical aliphatique qui contient de 1 à 6 atomes de carbone et qui peut porter un alcoxy en $C_1-C_6$ ou un aryloxy en $C_6-C_{12}$,
- un radical cycloaliphatique contenant de 5 à 8 atomes de carbone,
- un radical bicyclo-aliphatique contenant de 7 à 14 atomes de carbone,
- un radical tricycloaliphatique contenant de 7 à 16 atomes de carbone,
- un radical aryle qui contient de 6 à 12 atomes de carbone et qui peut porter un, deux ou trois substituants pris dans l'ensemble constitué par les halogènes, les alkyles en $C_1-C_4$ et les alcoxy en $C_1-C_4$,
- un radical aralkyle ou aralcényle contenant de 7 à 13 atomes de carbone,
- un radical alcoxy contenant de 1 à 6 atomes de carbone,
- un radical cycloalcoxy contenant de 5 à 8 atomes de carbone,
- un radical bicycloalcoxy contenant de 7 à 12 atomes de carbone,
- un radical tricycloalcoxy contenant de 7 à 16 atomes de carbone,
- un radical aryloxy contenant de 6 à 12 atomes de carbone,
- un radical pyridyle, pyrrolyle, imidazolyle, pyrazolyle, pyrazinyle, pyrimidinyle ou pyridazinyle, ou
- un radical alcoxycarbonyle contenant au total de 2 à 7 atomes de carbone,

$R^5$ représente un alkyle contenant de 1 à 6 atomes de carbone, un aryle contenant de 6 à 12 atomes de carbone, éventuellement porteur d'un méthyle ou d'un chlore, ou un dialkylamino contenant de 1 à 4 atomes de carbone dans chacun de ses radicaux alkyles,

$R^6$ et $R^7$ représentent chacun, indépendamment l'un de l'autre, un alkyle contenant de 1 à 6 atomes de carbone, un alcényle contenant de 3 à 6 atomes de carbone, un cycloalkyle contenant de 5 à 8 atomes de carbone, un bicycloalkyle contenant de 7 à 14 atomes de carbone, un tricycloalkyle contenant de 7 à 16 atomes de carbone, un aralkyle qui contient de 7 à 9 atomes de carbone et qui peut porter un méthyle ou un halogène, ou un radical hétérocyclique relié par une chaîne alkylène à l'atome d'azote du radical amino en position 3,

$R^6$ peut en outre représenter l'hydrogène,

$R^7$ peut en outre représenter un radical de formule:

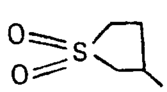

$R^8$ représente un alkyle contenant de 1 à 4 atomes de carbone,

$R^9$ représente un alkyle contenant de 1 à 6 atomes de carbone, un aryle en $C_6-C_{12}$ éventuellement porteur d'un méthyle ou d'un chlore, ou un dialkylamino dont chacun des alkyles contient de 1 à 4 atomes de carbone,

$R^{10}$ représente un alkyle contenant de 1 à 4 atomes de carbone et

$R^{11}$ représente un alkyle contenant de 1 à 6 atomes de carbone,

ou d'un sel d'une telle sydnonimine,

- d'un photostabilisant répondant à la formule générale II:

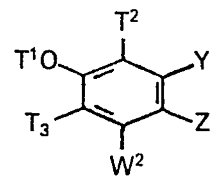

(II)

dans laquelle:

$T^1$ représente l'hydrogène, un alkyle à 1 ou 2 atomes de carbone, un hydroxy-2 éthyle ou un radical G,

$T^2$ représente l'hydrogène, un alkyle à 1 ou 2 atomes de carbone ou un alcanoyloxy contenant de 1 à 4 atomes de carbone,

$T^3$ représente l'hydrogène ou un alkyle à 1 ou 2 atomes de carbone,

Y représente un radical hydroxy, un alcoxy à 1 ou 2 atomes de carbone ou un hydroxy-2 éthoxy et

Z représente un radical de formule:

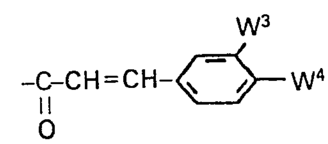

ou

Y et Z forment ensemble un élément structural répondant à l'une des formules III, IV et V:

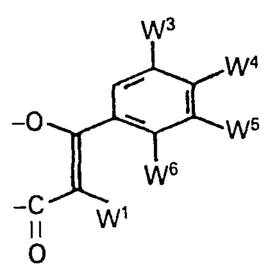

(III)

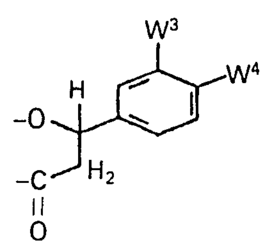

(IV)

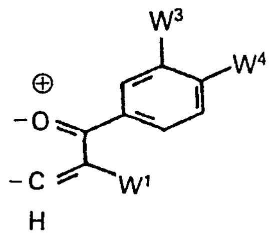

(V)

dont la valence libre qui part de l'oxygène est liée à la position de Y et dont la valence libre qui part du carbone est liée à la position de Z,

$W^1$ représente l'hydrogène, un radical –OH, un alcoxy à 1 ou 2 atomes de carbone ou un radical –OG,

$W^2$ représente l'hydrogène ou un radical –OH ou –OG,

$W^3$ représente l'hydrogène, un radical –OH, un alcoxy à 1 ou 2 atomes de carbone ou un hydroxy-2 éthoxy,

$W^4$ représente l'hydrogène, un radical –OH, un alcoxy à 1 ou 2 atomes de carbone ou un hydroxy-2 éthoxy,

$W^5$ représente l'hydrogène ou un radical –OH,

$W^6$ représente l'hydrogène ou un radical –OH et

G représente le radical d'un monosaccharide ou d'un disaccharide, et/ou des esters de ces composés avec des acides ainsi que, le cas échéant, des sels de ces composés avec des bases,

– et éventuellement d'autres adjuvants et additifs, à l'exception d'un mélange de molsidomines et d'un ou plusieurs photostabilisants répondant à la formule générale VI:

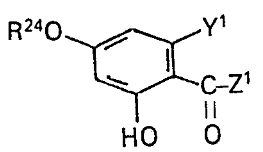

(VI)

dans laquelle:

$Y^1$ représente un radical hydroxy, un alcoxy contenant un ou deux atomes de carbone ou un hydroxy-2 éthoxy,

$Z^1$ représente un radical de formule:

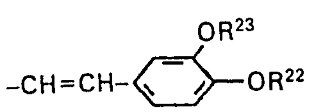

ou

$Y^1$ et $Z^1$ forment ensemble un élément structural répondant à l'une des formules VII et VIII:

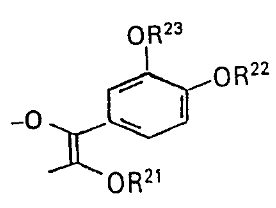

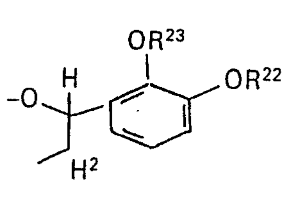

(VII) (VIII)

dont la valence libre partant de l'oxygène est lié à la position de $Y^1$ et dont la valence libre partant du carbone est liée à la position de $Z^1$,

$R^{21}$ représente l'hydrogène ou un radical $G^1$,

$R^{22}$ représente l'hydrogène, un alkyle à 1 ou 2 atomes de carbone ou un hydroxy-2 éthyle,

$R^{23}$ représente l'hydrogène, un alkyle à 1 ou 2 atomes de carbone ou un hydroxy-2 éthyle,

$R^{24}$ représente l'hydrogène, un alkyle à 1 ou 2 atomes de carbone, un hydroxy-2 éthyle ou un radical $G^1$ et

$G^1$ représente le radical d'un monosaccharide ou d'un disaccharide, ou d'un ester d'un composé de formule VI avec un acide acceptable du point de vue pharmacologique, ainsi qu'éventuellement d'un de ses sels formés avec des bases acceptables du point de vue pharmacologique.

2. Mélange photostable selon la revendication 1, caractérisé en ce que la sydnonimine répond à la formule générale Ia:

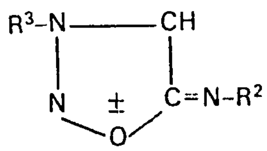

(Ia)

dans laquelle:

$R^2$ représente l'hydrogène ou un radical $-COR^4$,

$R^3$ représente l'un des radicaux:

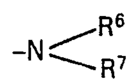

ou

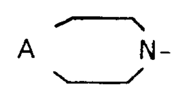

A représente l'oxygène ou l'un des radicaux:

$\geq S(O)_m$, $\geq N-COOR^8$ et $\geq N-SO_2R^9$

m est égal à 0, à 1 ou à 2,

$R^4$ représente l'hydrogène, un alkyle en $C_1-C_4$ éventuellement porteur d'un alcoxy en $C_1-C_4$, un cycloalkyle contenant de 5 à 8 atomes de carbone, un bicycloalkyle contenant de 7 à 12 atomes de carbone, un phényle éventuellement porteur d'un halogène, un alcoxy contenant de 4 atomes de carbone, ou un cycloalcoxy contenant de 5 à 8 atomes de carbone,

$R^6$ représente l'hydrogène ou un alkyle contenant de 1 à 4 atomes de carbone,

$R^7$ représente un radical de formule:

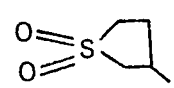

$R^8$ représente un alkyle contenant de 1 à 4 atomes de carbone et

$R^9$ représente un alkyle contenant de 1 à 4 atomes de carbone, ou un radical dialkylamino dont chacun des alkyles contient de 1 à 4 atomes de carbone.

3. Composition pharmaceutique photostabilisée qui est constituée:

– d'une substance active répondant à la formule générale I:

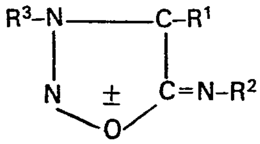

(I)

dans laquelle $R^1$, $R^2$ et $R^3$ ont les significations qui leur ont été données à la revendication 1, ou d'un de ses sels acceptables du pont de vue pharmacologique,

– d'un photostabilisant répondant à la formule II définie à la revendication 1, ou d'un ester formé par un tel photostabilisant avec un acide acceptable du point de vue pharmacologique, ainsi qu'éventuellement d'un de ses sels formés avec des bases acceptables du point de vue pharmacologique,

– et éventuellement aussi d'autres adjuvants et additifs usuels dans des compositions pharmaceutiques.

4. Composition pharmaceutique photostabilisée selon la revendication 3, qui est constituée:

– d'une substance active répondant à la formule générale Ia:

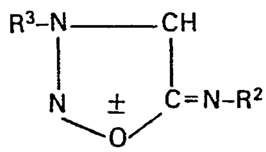

(Ia)

dans laquelle $R^2$ et $R^3$ ont les significations qui leur ont été données à la revendication 2, ou d'un de ses sels acceptables du point de vue pharmacologique,

– d'un photostabilisant répondant à la formule II définie à la revendication 2, ou d'un ester formé par un tel photostabilisant avec un acide acceptable du point de vue pharmacologique, ainsi qu'éventuellement un de ses sels formés avec une base acceptable du point de vue pharmacologique,

– et éventuellement aussi d'autres adjuvants et additifs usuels dans des compositions pharmaceutiques.

5. Mélange photostabilisé ou composition pharmaceutique stabilisée à base d'une sydnonimine selon l'une quelconque des revendications 1 à 4, caractérisés en ce que le photostabilisant est un composé de formule II dans lequel:

Y, Z et, dans la mesure où ils existent, $W^1$ et G ont les significations qui leur ont été données à la revendication 1,

$W^3$ représente un hydroxy ou un hydroxy-2 éthoxy,

$W^4$ représente un radical –OH, un alcoxy à 1 ou 2 atomes de carbone ou un hydroxy-2 éthoxy,

$T^1$ représente l'hydrogène, un hydroxy-2 éthyle ou G $T^2$ et $T^3$ représentent chacun l'hydrogène, ou un ester d'un tel composé et éventuellement l'un de ses sels.

6. Mélange photostabilisé ou composition pharmaceutique stabilisée à base d'une sydnonimine selon l'une quelconque des revendications 1 à 4, caractérisés en ce que le photostabilisant est l'hespéridine, l'hespérétine, la quercétine ou l'un de ses dérivés, la rutine, la naringénine, le camphérol ou l'un de ses dérivés, plus particulièrement son éther diméthylique en 7,4', la morine, l'apigétrine, la lutéoline ou son glucoside en 7, ou la troxérutine, ou encore un ester d'un de ses composés ainsi qu'éventuellement l'un de leurs sels.

7. Mélange photostabilisé ou composition pharmaceutique stabilisée à base d'une sydnonimine selon l'une quelconque des revendications 1 à 4, caractérisé en ce que la molsidomine et le photostabilisant s'y trouvent dans un rapport pondéral compris entre 1:0,001 et 1:50.

8. Procédé pour photostabiliser des sydnonimines répondant à la formule générale I:

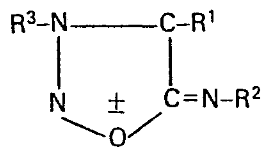

(I)

dans laquelle $R^1$, $R^2$ et $R^3$ ont les significations qui leur ont été données à la revendication 1, procédé caractérisé en ce qu'on mélange soigneusement et homogénéise un tel composé avec un photostabilisant répondant à la formule générale II:

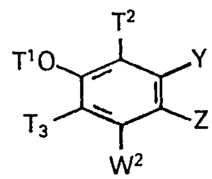

(II)

dans laquelle $T^1$ à $T^3$, $W^2$, Y et Z ont les significations qui leur ont été données à la revendication 1, et/ou un ester formé par un tel composé avec un acide, ainsi qu'éventuellement un sel d'un tel composé et d'une base, et éventuellement avec d'autres adjuvants et additifs.

9. Application de composés répondant à la formule générale II:

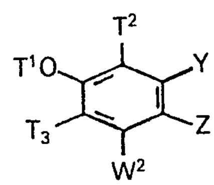

(II)

dans laquelle $T^1$ à $T^3$, $W^2$, Y et Z ont les significations qui leur ont été données à la revendication 1, et/ou d'un ester formé par un tel composé avec un acide ainsi qu'éventuellement l'un de ses sels, à la photostabilisation de sydnonimines répondant à la formule générale I:

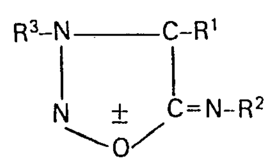

(I)

dans laquelle $R^1$, $R^2$ et $R^3$ ont les significations qui leur ont été données à la revendication 1, et de leur sels.

10. Application selon la revendication 9 de composés de formule II et de leurs esters dérivant d'acides acceptables du point de vue pharmacologique, ainsi qu'éventuellement de leurs sels formés avec des bases acceptables du point de vue pharmacologique, à la photostabilisation de sydnonimines de formule I ou Ia et de leurs sels acceptables du point de vue pharmacologique.

**Revendications pour l'Etat contractat AT**

1. Procédé pour photostabiliser une sydnonimine répondant à la formule générale I:

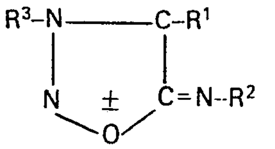

(I)

dans laquelle:

$R^1$ représente l'hydrogène, un alkyle contenant de 1 à 5 atomes de carbone ou un halogène,

$R^2$ représente l'hydrogène, $-NO$, $-COR^4$ ou $-SO_2R^5$,

$R^3$ représente l'un des radicaux suivants:

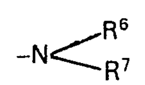

,

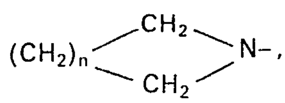

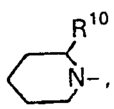

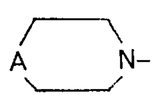

et

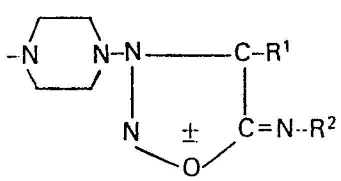

A représente l'oxygène ou un radical $\geq S(O)_m$, $\geq N-R^{11}$, $\geq N-COOR^8$ ou $\geq N-SO_2R^9$,

m est égal à 0, à 1 ou à 2,

n est égal à 2, à 3, à 4 ou à 5,

$R^4$ représente:

- l'hydrogène,
- un radical aliphatique qui contient de 1 à 6 atomes de carbone et qui peut porter un alcoxy en $C_1-C_6$ ou un aryloxy en $C_6-C_{12}$,
- un radical cycloaliphatique contenant de 5 à 8 atomes de carbone,
- un radical bicyclo-aliphatique contenant de 7 à 14 atomes de carbone,
- un radical tricycloaliphatique contenant de 7 à 16 atomes de carbone,
- un radical aryle qui contient de 6 à 12 atomes de carbone et qui peut porter un, deux ou trois substituants pris dans l'ensemble constitué par les halogènes, les alkyles en $C_1-C_4$ et les alcoxy en $C_1-C_4$,
- un radical aralkyle ou aralcényle contenant de 7 à 13 atomes de carbone,
- un radical alcoxy contenant de 1 à 6 atomes de carbone,
- un radical cycloalcoxy contenant de 5 à 8 atomes de carbone,
- un radical bicycloalcoxy contenant de 7 à 12 atomes de carbone,
- un radical tricycloalcoxy contenant de 7 à 16 atomes de carbone,
- un radical aryloxy contenant de 6 à 12 atomes de carbone,
- un radical pyridyle, pyrrolyle, imidazolyle, pyrazolyle, pyrazinyle, pyrimidinyle ou pyridazinyle, ou
- un radical alcoxycarbonyle contenant au total de 2 à 7 atomes de carbone,

$R^5$ représente un alkyle contenant de 1 à 6 atomes de carbone, un aryle contenant de 6 à 12 atomes de carbone, éventuellement porteur d'un méthyle ou d'un chlore, ou un dialkylamino contenant de 1 à 4 atomes de carbone dans chacun de ces radicaux alkyles,

$R^6$ et $R^7$ représentent chacun, indépendamment l'un de l'autre, un alkyle contenant de 1 à 6 atomes de carbone, un alcényle contenant de 3 à 6 atomes de carbone, un cycloalkyle contenant de 5 à 8 atomes de carbone, un bicycloalkyle contenant de 7 à 14 atomes de carbone, un tricycloalkyle contenant de 7 à 16 atomes de carbone, un aralkyle qui contient de 7 à 9 atomes de carbone et qui peut porter un méthyle ou un halogène, ou un radical hétérocyclique relié par une chaîne alkylène à l'atome d'azote du radical amino en position 3,

$R^6$ peut en outre représenter l'hydrogène,

$R^7$ peut en outre représenter un radical de formule:

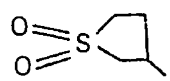

$R^8$ représente un alkyle contenant de 1 à 4 atomes de carbone,

$R^9$ représente un alkyle contenant de 1 à 6 atomes de carbone, un aryle en $C_6-C_{12}$ éventuellement porteur d'un méthyle ou d'un chlore, oú un dialkylamino dont chacun des alkyles contient de 1 à 4 atomes de carbone,

$R^{10}$ représente un alkyle contenant de 1 à 4 atomes de carbone et

$R^{11}$ représente un alkyle contenant de 1 à 6 atomes de carbone, ou d'un sel d'une telle sydnonimine, procédé caractérisé en ce qu'on mélange la sydnonimine ou l'un de ses sels avec un photostabilisant répondant à la formule générale II:

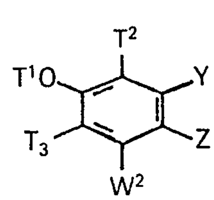

(II)

dans laquelle:

$T^1$ représente l'hydrogène, un alkyle à 1 ou 2 atomes de carbone, un hydroxy-2 éthyle ou un radical G,

$T^2$ représente l'hydrogène, un alkyle à 1 ou 2 atomes de carbone ou un alcanoyloxy contenant de 1 à 4 atomes de carbone,

$T^3$ représente l'hydrogène ou un alkyle à 1 ou 2 atomes de carbone,

Y représente un radical hydroxy, un alcoxy à 1 ou 2 atomes de carbone ou un hydroxy-2 éthoxy et

Z représente un radical de formule:

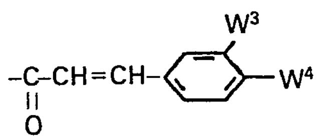

ou

Y et Z forment ensemble un élément structural répondant à l'une des formules III, IV et V:

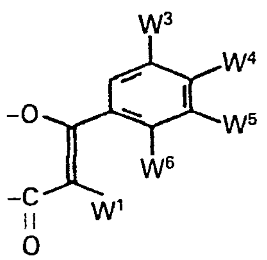

(III)

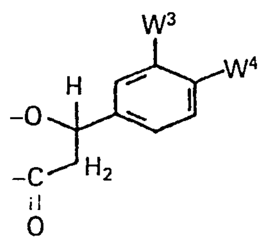

(IV)

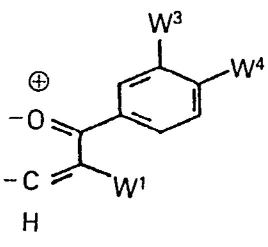

(V)

dont la valence libre qui part de l'oxygène est liée à la position de Y et dont la valence libre qui part du carbone est liée à la position de Z,

$W^1$ représente l'hydrogène, un radical -OH, un alcoxy à 1 ou 2 atomes de carbone ou un radical -OG,

$W^2$ représente l'hydrogène ou un radical -OH ou -OG,

$W^3$ représente l'hydrogène, un radical -OH, un alcoxy à 1 ou 2 atomes de carbone ou un hydroxy-2 éthoxy,

$W^4$ représente l'hydrogène, un radical -OH, un alcoxy à 1 ou 2 atomes de carbone ou un hydroxy-2 éthoxy,

$W^5$ représente l'hydrogène ou un radical -OH,

$W^6$ représente l'hydrogène ou un radical -OH et

G représente le radical d'un monosaccharide ou d'un disaccharide, et/ou un ester d'un tel composé avec un acide, ainsi qu'éventuellement un sel d'un tel composé et d'une base, et éventuellement avec d'autres adjuvants et additifs, sauf un procédé de photostabilisation de molsidomine et de mélanges de molsidomine et d'autres constituants, selon lequel on ajoute à la molsidomine ou à ses mélanges comme photostabilisant(s), un ou plusieurs composés répondant à la formule générale VI:

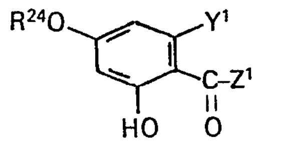

(VI)

dans laquelle:

$Y^1$ représente un radical hydroxy, un alcoxy contenant un ou deux atomes de carbone ou un hydroxy-2 éthoxy,

$Z^1$ représente un radical de formule:

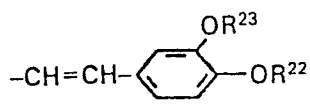

ou

$Y^1$ et $Z^1$ forment ensemble un élément structural répondant à l'une des formules VII et VIII:

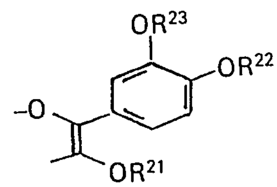

(VII)

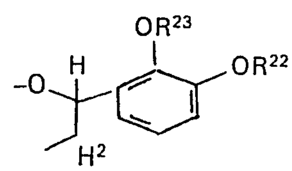

(VIII)

dont la valence libre partant de l'oxygène est lié à la position de $Y^1$ et dont la valence libre partant du carbone est liée à la position de $Z^1$,

$R^{21}$ représente l'hydrogène ou un radical $G^1$,

$R^{22}$ représente l'hydrogène, un alkyle à 1 ou 2 atomes de carbone ou un hydroxy-2 éthyle,

$R^{23}$ représente l'hydrogène, un alkyle à 1 ou 2 atomes de carbone ou un hydroxy-2 éthyle,

$R^{24}$ représente l'hydrogène, un alkyle à 1 ou 2 atomes de carbone, un hydroxy-2 éthyle ou un radical $G^1$ et

$G^1$ représente le radical d'un monosaccharide ou d'un disaccharide, ou un ester d'un composé de formule VI avec un acide acceptable du point de vue pharmacologique, ainsi qu'éventuellement l'un de ses sels avec un acide acceptable du point de vue pharmacologique.

2. Procédé selon la revendication 1 caractérisé en ce que la sydnonimine répond à la formule générale Ia:

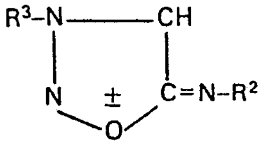

(Ia)

dans laquelle:

$R^2$ représente l'hydrogène ou un radical $-COR^4$,

$R^3$ représente l'un des radicaux:

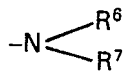

ou

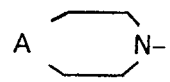

A représente l'oxygène ou l'un des radicaux:

$>S(O)_m$, $>N-COOR^8$ et $>N-SO_2R^9$

m est égal à 0, à 1 ou à 2,

$R^4$ représente l'hydrogène, un alkyle en $C_1-C_4$ éventuellement porteur d'un alcoxy en $C_1-C_4$, un cycloalkyle contenant de 5 à 8 atomes de carbone, un bicycloalkyle contenant de 7 à 12 atomes de carbone, un phényle éventuellement porteur d'un halogène, un alcoxy contenant de 1 à 4 atomes de carbone, ou un cycloalcoxy contenant de 5 à 8 atomes de carbone,

$R^6$ représente l'hydrogène ou un alkyle contenant de 1 à 4 atomes de carbone,

$R^7$ représente un radical de formule:

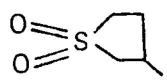

$R^8$ représente un alkyle contenant de 1 à 4 atomes de carbone et

$R^9$ représente un alkyle contenant de 1 à 4 atomes de carbone, ou un radical dialkylamino dont chacun des alkyles contient de 1 à 4 atomes de carbone.

3. Procédé selon la revendication 1 caractérisé en ce que, pour préparer des compositions pharmaceutiques photostabilisées, on utilise une substance active répondant à la formule générale I:

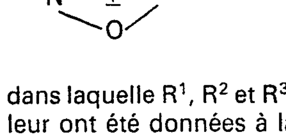

(I)

dans laquelle $R^1$, $R^2$ et $R^3$ ont les significations qui leur ont été données à la revendication 1, ou un sel pharmacologiquement acceptable d'une telle substance active, un photostabilisant répondant à la formule II définie à la revendication 1, ou un ester d'un tel photostabilisant avec un acide acceptable du point de vue pharmacologique, ainsi qu'éventuellement l'un de ses sels avec une base acceptable du point de vue pharmacologique, et éventuellement aussi d'autres adjuvants et additifs usuels dans des compositions pharmaceutiques.

4. Procédé selon la revendication 2 caractérisé en ce que, pour préparer des compositions pharmaceutiques photostabilisées, on utilise une substance active répondant à la formule générale Ia:

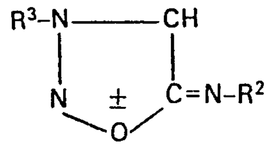

(Ia)

dans laquelle $R^2$ et $R^3$ ont les significations qui leur ont été données à la revendication 2, ou un sel pharmacologiquement acceptable d'une telle substance active, un photostabilisant répondant à la formule II définie à la revendication 2, ou un ester d'un tel photostabilisant avec un acide acceptable du point de vue pharmacologique, ainsi qu'éventuellement l'un de ses sels avec une base acceptable du point de vue pharmacologique, et éventuellement aussi d'autres adjuvants et additifs usuels dans des compositions pharmaceutiques.

5. Procédé selon l'une quelconque des revendications 1 à 4, caractérisé en ce qu'on utilise, comme photostabilisant, un composé de formule II dans lequel:

Y, Z et, dans la mesure où ils existent, $W^1$ et G ont les significations qui leur ont été données à la revendication 1,

$W^3$ représente un hydroxy ou un hydroxy-2 éthoxy,

$W^4$ représente un radical -OH, un alcoxy à 1 ou 2 atomes de carbone ou un hydroxy-2 éthoxy,

$T^1$ représente l'hydrogène, un hydroxy-2 éthyle ou G et

$T^2$ et $T^3$ représentent chacun l'hydrogène,

ou un ester d'un tel composé et éventuellement l'un de ses sels.

6. Procédé selon l'une quelconque des revendications 1 à 4, caractérisé en ce qu'on utilise, comme photostabilisant, l'hespéridine, l'hespérétine, la quercétine ou l'un de ses dérivés, la rutine, la naringénine, le camphérol ou l'un de ses dérivés, plus particulièrement son éther diméthylique en 7,4', la morine, l'apigétrine, la lutéoline ou son glucoside en 7, ou la troxérutine, ou encore un ester d'un de ses composés ainsi qu'éventuellement l'un de leurs sels.

7. Procédé selon l'une quelconque des revendications 1 à 4, caractérisé en ce qu'on met en jeu la molsidomine et un photostabilisant dans un rapport pondéral compris entre 1:0,001 et 1:50.

8. Procédé selon l'une quelconque des revendications 1 à 7, caractérisé en ce qu'on mélange soigneusement et homogénéise les composantes.

9. Application de composés répondant à la formule générale II:

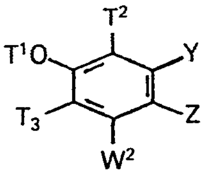

(II)

dans laquelle $T^1$ à $T^3$, $W^2$ Y et Z ont les significations qui leur ont été données à la revendication 1, et/ou d'esters de ces composés avec des acides, ainsi qu'éventuellement de leurs sels, à la photostabilisation de sydnonimines répondant à la formule générale I:

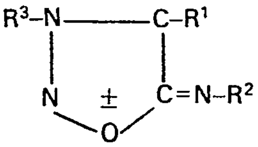

(I)

dans laquelle $R^1$, $R^2$ et $R^3$ ont les significations qui leur ontété données à la revendication 1, et de leurs sels.

10. Application selon la revendication 9 de composés de formule II et de leurs esters dérivant d'acides acceptables du point de vue pharmacologique, ainsi qu'éventuellement de leurs sels formés avec des bases acceptables du point de vue pharmacologique, à la photostabilisation de sydnonimines de formule I ou Ia et de leurs sels acceptables du point de vue pharmacologique.

**Claims for the Contracting States: BE, CH, DE, FR, GB, IT, LI, NL**

1. Photostable mixture of a sydnone imine of the general formula I

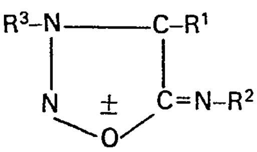

(I)

wherein

$R^1$ denotes hydrogen, alkyl having 1 to 5 C atoms or halogen,

$R^2$ denotes hydrogen, $-NO$, $-COR^4$ or $-SO_2R^5$ and

$R^3$ denotes one of the radicals

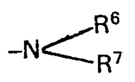 , 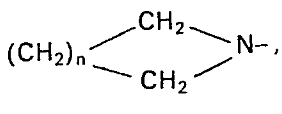

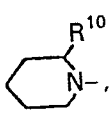 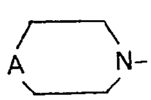 or

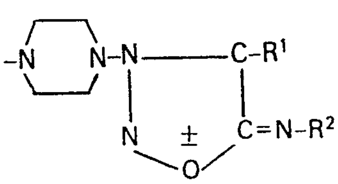

wherein

A represents oxygen or one of the groups $>S(O)_m$, $>N-R^{11}$, $>N-COOR^8$ or $>N-SO_2R^9$ and

m represents 0, 1 or 2 and

n represents 2, 3, 4 or 5, and

$R^4$ denotes hydrogen, an aliphatic radical which has 1 to 6 C atoms and which can also be substituted by alkoxy having 1 to 6 C atoms or araloxy having 6 to 12 C atoms, a cycloaliphatic radical having 5 to 8 C atoms, a bicycloaliphatic radical having 7 to 14 C atoms, a tricycloaliphatic radical having 7 to 16 C atoms, aryl which has 6 to 12 C atoms and which can also be monosubstituted, disubstituted or trisubstituted by halogen and/or alkyl having 1 to 4 C atoms and/or alkoxy having 1 to 4 C atoms, aralkyl or aralkenyl having 7 to 13 C atoms, alkoxy having 1 to 6 C atoms, cycloalkoxy having 5 to 8 C atoms, bicycloalkoxy having 7 to 12 C atoms, tricycloalkoxy having 7 to 16 C atoms, aryloxy having 6 to 12 C atoms, pyridyl, pyrrolyl, imidazolyl, pyrazolyl, pyrazinyl, pyrimidinyl, pyridazinyl or alkoxycarbonyl having a total of 2 to 7 C atoms,

$R^5$ denotes alkyl having 1 to 6 C atoms or aryl which has 6 to 12 C atoms and which can also be substituted by methyl or chlorine, or $R^5$ denotes a dialkylamino group having 1 to 4 C atoms in each of the alkyl radicals,

$R^6$ and $R^7$ independently of one another denote alkyl having 1 to 6 C atoms, alkenyl having 3 to 6 C atoms, cycloalkyl having 5 to 8 C atoms, bicycloalkyl having 7 to 14 C atoms, tricycloalkyl having 7 to 16 C atoms or aralkyl having 7 to 9 C atoms, it being possible for the aralkyl group to be substituted by methyl or halogen, or denote a heterocyclic ring attached via an alkylene chain to the nitrogen atom of the 3-amino group, and

$R^6$ additionally can be hydrogen and

$R^7$ additionally can be a radical of the formula

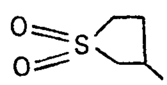

$R^8$ denotes alkyl having 1 to 4 C atoms,

$R^9$ denotes alkyl having 1 to 6 C atoms or aryl which has 6 to 12 C atoms and can also be substituted by methyl or chlorine, or

$R^9$ denotes a dialkylamino group having 1 to 4 C atoms in each of the alkyl radicals,

$R^{10}$ denotes alkyl having 1 to 4 C atoms and

$R^{11}$ denotes alkyl having 1 to 6 C atoms, or a salt of the sydnone imine, a photostabilizer of the general f ormula II

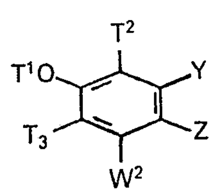

(II)

wherein $T^1$ denotes hydrogen, alkyl having 1 or 2 C atoms, β-hydroxyethyl or a radical G,

$T^2$ denotes hydrogen, alkyl having 1 or 2 C atoms or alkanoyloxy having 1 to 4 C atoms,

$T^3$ denotes hydrogen or alkyl having 1 or 2 C atoms,

Y denotes a hydroxyl group, alkoxy having 1 or 2 C atoms or β-hydroxyethoxy and

Z denotes a group of the formula

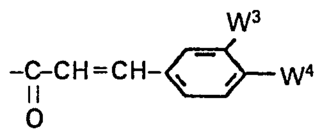

or

Y and Z together denote a structural element of the formulae III, IV or V

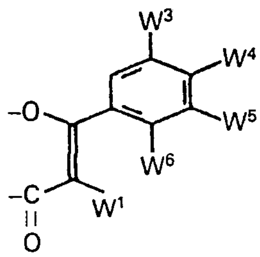

(III)

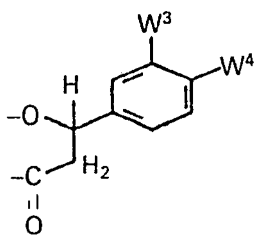

(IV)

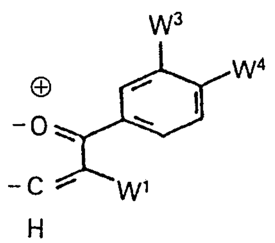

(V)

in which the free oxygen valency is attached in the Y-position and the free carbon valency is attached in the Z-position, and

$W^1$ is hydrogen or one of the groups –OH, alkoxy having 1 or 2 C atoms or –OG,

$W^2$ is hydrogen or one of the groups –OH and –OG,

$W^3$ is hydrogen or one of the groups –OH, alkoxy having 1 or 2 C atoms and β-hydroxyethoxy,

$W^4$ is hydrogen or one of the groups –OH, alkoxy having 1 or 2 C atoms and β-hydroxyethoxy,

$W^5$ is hydrogen or –OH,

$W^6$ is hydrogen or –OH and

G is the radical of a monosaccharide or disaccharide, and/or an ester of these compounds with acids and also, if appropriate, a salt thereof with bases, and, if appropriate, further auxiliaries and additives, with the exception of a mixture of molsidomine and one or more photostabilizers of the general formula VI

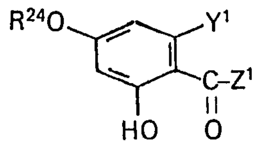

(VI)

wherein

$Y^1$ is a hydroxyl group, alkoxy having 1 or 2 C atoms or β-hydroxyethoxy and

$Z^1$ is a group of the formula

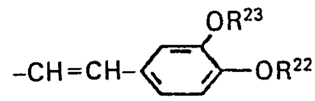

or $Y^1$ and $Z^1$ together denote a structural element of the formula VII or VIII

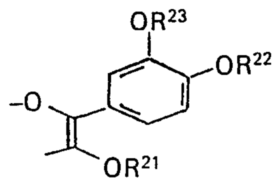

(VII)

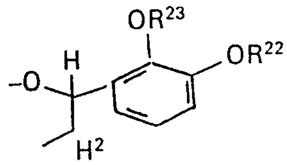

(VIII)

the free oxygen valency of which is attached in the $Y^1$-position and the free carbon valency is attached in the $Z^1$-position and

$R^{21}$ is hydrogen or a radical $G^1$,

$R^{22}$ is hydrogen, alkyl having 1 or 2 C atoms or β-hydroxyethyl,

$R^{23}$ is hydrogen, alkyl having 1 or 2 C atoms or β-hydroxyethyl,

$R^{24}$ is hydrogen, alkyl having 1 or 2 C atoms or β-hydroxyethyl or a radical $G^1$ and

$G^1$ is the radical of a monosaccharide or disaccharide, or an ester of the compound of the formula VI with a pharmacologically acceptable acid as well as, if appropriate, its salt with a pharmacologically acceptable base.

2. Photostable mixture according to Claim 1, characterized in that the sydnone imine corresponds to the general formula Ia

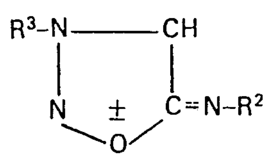

(Ia)

wherein

$R^2$ denotes hydrogen or $-COR^4$, and

$R^3$ denotes one of the radicals

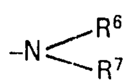

or

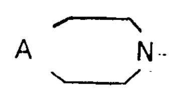

wherein A represents oxygen or one or the groups $>S(O)_m$, $>N\text{-}COOR^8$ or $>N\text{-}SO_2R^9$ and

m represents 0, 1 or 2 and

$R^4$ denotes hydrogen, alkyl which has 1 to 4 C atoms and which can also be substituted by alkoxy having 1 to 4 C atoms, cycloalkyl having 5 to 8 C atoms, bicycloalkyl having 7 to 12 C atoms, phenyl which can also be substituted by halogen, alkoxy having 1 to 4 C atoms, or cycloalkoxy having 5 to 8 C atoms,

$R^6$ denotes hydrogen or alkyl having 1 to 4 C atoms,

$R^7$ denotes a radical of the formula

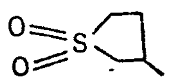

$R^8$ denotes alkyl having 1 to 4 C atoms and

$R^9$ denotes alkyl having 1 to 4 C atoms or a dialkylamino group having 1 to 4 C atoms in each of the alkyl radicals.

3. Photostabilized pharmaceutical formulation consisting of an active compound of the general formula I

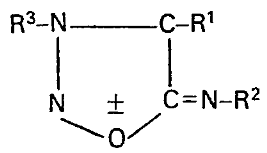

(I)

wherein $R^1$, $R^2$ and $R^3$ have the meanings indicated in Claim 1, or a pharmaceutically acceptable salt of the active compound, a photostabilizer of the formula II indicated in Claim 1 or an ester of this photostabilizer with pharmaceutically acceptable acids and, if appropriate, a salt thereof with pharmaceutically acceptable bases and, if appropriate, further auxiliaries and additives customary in pharmaceutical formulations.

4. Photostabilized pharmaceutical formulation according to Claim 3, consisting of an active compound of the general formula Ia

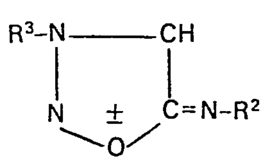

(Ia)

wherein $R^2$ and $R^3$ have the meanings indicated in Claim 2, or a pharmaceutically acceptable salt of the active compound, a photostabilizer of the formula II indicated in Claim 2 or an ester of this photostabilizer with pharmaceutically acceptable acids and, if appropriate, a salt thereof with pharmaceutically acceptable bases and, if appropriate, further auxiliaries and additives customary in pharmaceutical formulations.

5. Photostabilized mixture or pharmaceutical formulation of a sydnone imine according to Claims 1 to 4, characterized in that the photostabilizer is a compound of the formula II wherein Y, Z and, if present, $W^1$ and G have the meanings mentioned in Claim 1,

$W^3$ denotes hydroxyl or β-hydroxyethoxy and

$W^4$ denotes one of the groups -OH, alkoxy having 1 or 2 C atoms and β-hydroxyethoxy,

$T^1$ denotes hydrogen, β-hydroxyethyl or G, and

$T^2$ and $T^3$ denote hydrogen, or an ester of these compounds and, if appropriate, a salt thereof.

6. Photostabilized mixture or pharmaceutical formulation of a sydnone imine according to Claims 1 to 4, characterized in that the photostabilizer is hesperidin, hesperetin, quercetin and derivatives thereof, rutin, naringenin, kaempferol and derivatives thereof, in particular the 7,4'-dimethyl ether thereof, morin, apigetrin, luteolin and the 7-glucoside thereof or troxerutin or an ester of these compounds and, if appropriate, a salt thereof.

7. Photostabilized mixture or pharmaceutical formulation of a sydnone imine according to Claims 1 to 4, characterized in that molsidomin and the photostabilizer are present in a ratio by weight of 1:0,001 to 1:50.

8. Process for photostabilizing sydnone imines of the general formula I

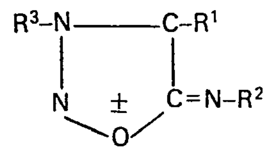

(I)

wherein

$R^1$, $R^2$ and $R^3$ have the meanings indicated in Claim 1, characterized in that the sydnone imine is thouroughly mixed and homogenized with a photostabilizer of the general formula II

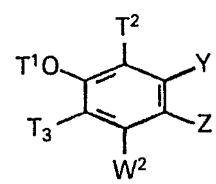

(II)

wherein $T^1$ to $T^3$, $W^2$, Y and Z have the meanings indicated in Claim 1, and/or an ester of these compounds with acids and, if appropriate, a salt thereof with bases and, if appropriate, further auxiliaries and additives.

9. The use of compounds of the general formula II

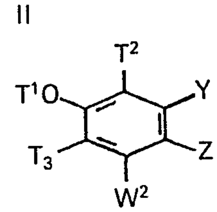

(II)

wherein

$T^1$ to $T^3$, $W^2$, Y and Z have the meanings indicated in Claim 1, and/or an ester of these compounds with acids and, if appropriate, a salt thereof, for photostabilizing sydnone imines of the general formula I

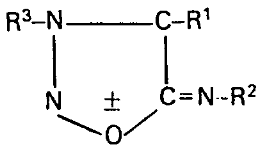

(I)

wherein

$R^1$, $R^2$ and $R^3$ have the meanings indicated in Claim 1, and salts thereof.

10. Use, according to Claim 9, of compounds of the formula II and esters thereof with pharmacologically acceptable acids and, if appropriate salts thereof with pharmacologically acceptable bases for photostabilizing sydnone imines of the formula I or Ia and pharmacologically acceptable salts thereof.

**Claims for the Contracting State AT:**

1. Process for stabilizing a sydnone imine of the general formula I

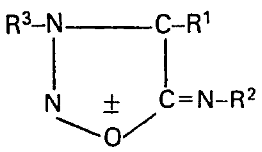

(I)

wherein

$R^1$ denotes hydrogen, alkyl having 1 to 5 C atoms or halogen,

$R^2$ denotes hydrogen, –NO, $-COR^4$ or $-SO_2R^5$ and

$R^3$ denotes one of the radicals

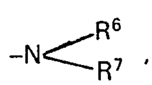
,
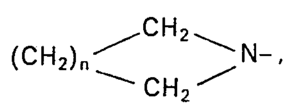

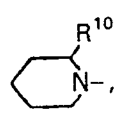
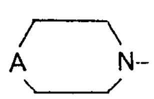
or

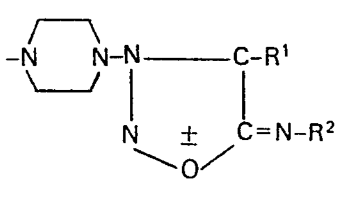

wherein

A represents oxygen or one of the groups >S(O)$_m$, >N–$R^{11}$, >N–$COOR^8$ or >N–$SO_2R^9$ and

m represents 0, 1 or 2 and

n represents 2, 3, 4 or 5, and

$R^4$ denotes hydrogen, an aliphatic radical which has 1 to 6 C atoms and which can also be substituted by alkoxy having 1 to 6 C atoms or aryloxy having 6 to 12 C atoms, a cycloaliphatic radical having 5 to 8 C atoms, a bicycloaliphatic radical having 7 to 14 C atoms, a tricycloaliphatic radical having 7 to 16 C atoms, aryl which has 6 to 12 C atoms and which can also be monosubstituted, disubstituted or trisubstituted by halogen and/or alkyl having 1 to 4 C atoms and/or alkoxy having 1 to 4 C atoms, aralkyl or aralkenyl having 7 to 13 C atoms, alkoxy having 1 to 6 C atoms, cycloalkoxy having 5 to 8 C atoms, bicycloalkoxy having 7 to 12 C atoms, tricycloalkoxy having 7 to 16 C atoms, aryloxy having 6 to 12 C atoms, pyridyl, pyrrolyl, imidazolyl, pyrazolyl, pyrazinyl, pyrimidinyl, pyridazinyl or alkoxycarbonyl having a total of 2 to 7 C atoms,

$R^5$ denotes alkyl having 1 to 6 C atoms or aryl which has 6 to 12 C atoms and which can also be substituted by methyl or chlorine, or $R^5$ denotes a dialkylamino group having 1 to 4 C atoms in each of the alkyl radicals,

$R^6$ and $R^7$ independently of one another denote alkyl having 1 to 6 C atoms, alkenyl having 3 to 6 C atoms, cycloalkyl having 5 to 8 C atoms, bicycloalkyl having 7 to 14 C atoms, tricycloalkyl having 7 to 16 C atoms or aralkyl having 7 to 9 C atoms, it being possible for the aralkyl group to be substituted by methyl or halogen, or denote a heterocyclic ring attached via an alkylene chain to the nitrogen atom of the 3-amino group, and

$R^6$ additionally can be hydrogen and

$R^7$ additionally can be a radical of the formula

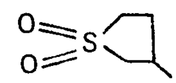

$R^8$ denotes alkyl having 1 to 4 C atoms,

$R^9$ denotes alkyl having 1 to 6 C atoms or aryl which has 6 to 12 C atoms and can also be substituted by methyl or chlorine, or

$R^9$ denotes a dialkylamino group having 1 to 4 C atoms in each of the alkyl radicals,

$R^{10}$ denotes alkyl having 1 to 4 C atoms and

$R^{11}$ denotes alkyl having 1 to 6 C atoms,

or a salt of the sydnone imine, a photostabilizer of the general formula II

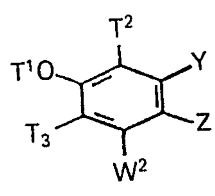

(II)

wherein $T^1$ denotes hydrogen, alkyl having 1 or 2 C atoms, β-hydroxyethyl or a radical G,

$T^2$ denotes hydrogen, alkyl having 1 or 2 C atoms or alkanoyloxy having 1 to 4 C atoms,

$T^3$ denotes hydrogen or alkyl having 1 or 2 C atoms,

Y denotes a hydroxyl group, alkoxy having 1 or 2 C atoms or β-hydroxyethoxy and

Z denotes a group of the formula

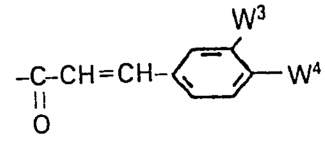

or

Y and Z together denote a structural element of the formulae III, IV or V

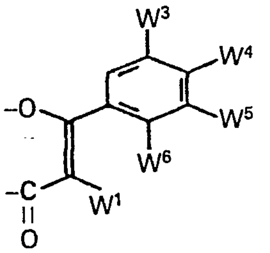

(III)

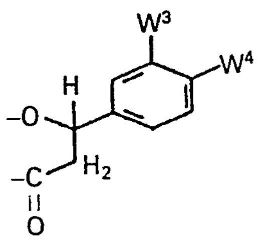

(IV)

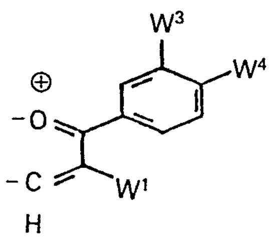

(V)

in which the free oxygen valency is attached in the Y-position and the free carbon valency is attached in the Z-position, and

$W^1$ is hydrogen or one of the groups –OH, alkoxy having 1 or 2 C atoms or –OG,

$W^2$ is hydrogen or one of the groups –OH and –OG,

$W^3$ is hydrogen or one of the groups –OH, alkoxy having 1 or 2 C atoms and β-hydroxyethoxy,

$W^4$ is hydrogen or one of the groups –OH, alkoxy having 1 or 2 C atoms and β-hydroxyethoxy,

$W^5$ is hydrogen or –OH,

$W^6$ is hydrogen or –OH and

G is the radical of a monosaccharide or disaccharide, and/or an ester of these compounds with acids and also, if appropriate, a salt thereof with bases, and, if appropriate, further auxiliaries and additives, with the exception of a process for photostabilizing molsidomine and of mixtures of molsidomine with mixing components wherein one or more compounds of the general formula VI

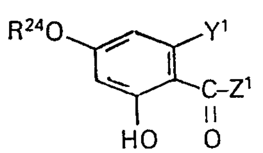

(VI)

are added to the molsidoine or its mixtures ad photostabilizer, in which formula

$Y^1$ is a hydroxyl group, alkoxy having 1 or 2 C atoms or β-hydroxyethoxy and

$Z^1$ is a group of the formula

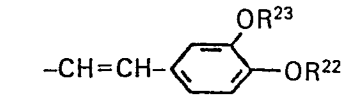

or $Y^1$ and $Z^1$ together denote a structural element of the formula VII or VIII

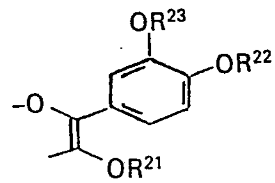

(VII)

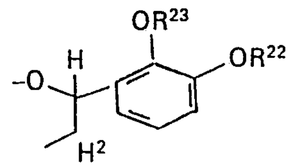

(VIII)

the free oxygen valency of which is attached in the $Y^1$-position and the free carbon valency is attached in the $Z^1$-position and

$R^{21}$ is hydrogen or a radical $G^1$,

$R^{22}$ is hydrogen, alkyl having 1 or 2 C atoms or β-hydroxyethyl,

$R^{23}$ is hydrogen, alkyl having 1 or 2 C atoms or β-hyroxyethyl,

$R^{24}$ is hydrogen, alkyl having 1 or 2 C atoms or β-hydroxyethyl or a radical $G^1$ and

$G^1$ is the radical of a monosaccharide or disaccharide, or an ester of the compound of the formula VI with a pharmacologically acceptable acid as well as, if appropriate, its salt with a pharmacologically acceptable base.

2. The process of Claim 1, characterized in that the sydnone imine corresponds to the general formula Ia

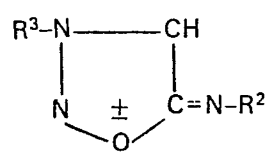

(Ia)

wherein

$R^2$ denotes hydrogen or $-COR^4$, and

$R^3$ denotes one of the radicals

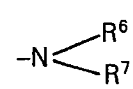

or

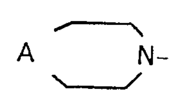

wherein A represents oxygen or one of the groups $>S(O)_m$, $>N-COOR^8$ or $>N-SO_2R^9$ and

m represents 0, 1 or 2 and

$R^4$ denotes hydrogen, alkyl which has 1 to 4 C atoms and which can also be substituted by alkoxy having 1 to 4 C atoms, cycloalkyl having 5 to 8 C atoms, bicycloalkyl having 7 to 12 C atoms, phenyl which can also be substituted by halogen, alkoxy having 1 to 4 C atoms, or cycloalkoxy having 5 to 8 C atoms,

$R^6$ denotes hydrogen or alkyl having 1 to 4 C atoms,

$R^7$ denotes a radical of the formula

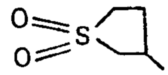

$R^8$ denotes alkyl having 1 to 4 C atoms and

$R^9$ denotes alkyl having 1 to 4 C atoms or a dialkylamino group having 1 to 4 C atoms in each of the alkyl radicals.

3. The process of Claim 1, characterized in that, for preparing photostabilized pharmaceutical preparations, there is used an active compound of the general formula I

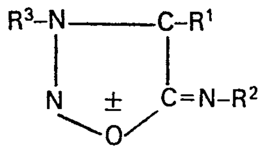

(I)

wherein $R^1$, $R^2$ and $R^3$ have the meanings indicated in Claim 1, or a pharmaceutically acceptable salt of the active compound, a photostabilizer of the formula II indicated in Claim 1 or an ester of this photostabilizer with pharmaceutically acceptable acids and, if appropriate, a salt thereof with pharmaceutically acceptable bases and, if appropriate, further auxiliaries and additives customary in pharmaceutical formulations.

4. The process of Claim 2, characterized in that, for preparing photostabilized pharmaceutical preparations, there is used an active compound of the general formula Ia

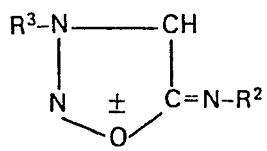

(Ia)

wherein $R^2$ and $R^3$ have the meanings indicated in Claim 2, or a pharmaceutically acceptable salt of the active compound, a photostabilizer of the formula II indicated in Claim 2 or an ester of this photostabilizer with pharmaceutically acceptable acids and, if appropriate, a salt thereof with pharmaceutically acceptable bases and, if appropriate, further auxiliaries and additives customary in pharmaceutical formulations.

5. The process of Claims 1 to 4, characterized in that the photostabilizer used is a compound of the formula II wherein Y, Z and, if present, $W^1$ and G have the meanings mentioned in Claim 1,

$W^3$ denotes hydroxyl or β-hydroxyethoxy and

$W^4$ denotes one of the groups –OH, alkoxy having 1 or 2 C atoms and β-hydroxyethoxy,

$T^1$ denotes hydrogen, β-hydroxyethyl or G, and

$T^2$ and $T^3$ denote hydrogen, or an ester of these compounds and, if appropriate, a salt thereof.

6. The process of Claims 1 to 4, characterized in that the photostabilizer used is hesperidin, hesperetin, quercetin and derivatives thereof, rutin, naringenin, kaempferol and derivatives thereof, in particular the 7,4'-dimethyl ether thereof, morin, apigetrin, luteolin and the 7-glucoside thereof or troxerutin or an ester of these compounds and, if appropriate, a salt thereof.

7. The process of Claims 1 to 4, characterized in that molsidomin and a photostabilizer are used in a ratio by weight of 1:0.001 to 1:50.

8. The process of Claims 1 to 7, characterized in that the components are thoroughly mixed and homogenized.

9. The use of compounds of the general formula II

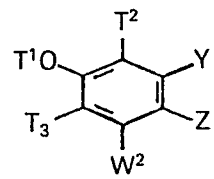

(II)

wherein

$T^1$ to $T^3$, $W^2$, Y and Z have the meanings indicated in Claim 1, and/or an ester of these compounds with acids and, if appropriate, a salt thereof, for photostabilizing sydnone imines of the general formula I

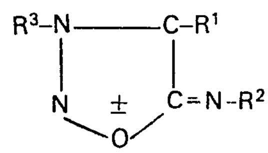

(I)

wherein

$R^1$, $R^2$ and $R^3$ have the meanings indicated in Claim 1, and salts thereof.

10. Use, according to Claim 9, of compounds of the formula II and esters thereof with pharmacologically acceptable acids and, if appropriate salts thereof with pharmacologically acceptable bases for photostabilizing sydnone imines of the formula I or Ia and pharmacologically acceptable salts thereof.